# EUROPÄISCHE PATENTSCHRIFT

(11) **EP 1 194 589 B1**
(45) Veröffentlichungstag und Bekanntmachung des Hinweises auf die Patenterteilung: **29.10.2008**
(21) Anmeldenummer: 00938996.6
(22) Anmeldetag: 07.07.2000
(51) Int. Cl.: C12Q 1/68

(54) **DNA-POLYMORPHISMEN IN STEROL-REGULATOR ELEMENT-BINDENDEN PROTEINEN**
DNA POLYMORPHISMS IN STEROL REGULATOR ELEMENT BINDING PROTEINS
POLYMORPHISMES DANS L'ADN DE PROTEINES DE FIXATION A UN ELEMENT REGULATEUR DE STEROL

(30) Priorität: 09.07.1999 CH 127799
(43) Veröffentlichungstag der Anmeldung: 10.04.2002
(73) Patentinhaber: Miserez, André R., 4147 Aesch (CH)
(72) Erfinder: Miserez, André R., 4147 Aesch (CH)
(74) Vertreter: Blum, Rudolf Emil
(86) Internationale Anmeldenummer: PCT/IB2000/000918
(87) Internationale Veröffentlichungsnummer: WO 2001/004352

(56) Entgegenhaltungen:
- US-A- 5 891 631
- MISEREZ ANDREW G ET AL: "Structure of the human gene encoding sterol regulatory element binding protein 2 (SREBF2)." GENOMICS, Bd. 40, Nr. 1, 1997, Seiten 31-40, XP002159138 ISSN: 0888-7543 in der Anmeldung erwähnt
- MISEREZ: "Die Bedeutung genetischer Faktoren bei der Entstehung des Herzinfarkts" UNI NOVA, WISSENSCHAFTMAGAZIN DER UNIVERSITÄT BASEL, [Online] Bd. 81, April 1998 (1998-04), XP002159139 Gefunden im Internet: <URL:http://www.zuv.unibas.ch/uni_nova/081 /11.shtml> [gefunden am 2001-01-30] in der Anmeldung erwähnt
- HUA XIANXIN ET AL: "Structure of the Human Gene Encoding Sterol Regulatory Element Binding Protein-1 (SREBF1) and Localization of SREBF1 and SREBF2 to Chromosomes 17p11.2 and 22q13." GENOMICS, Bd. 25, Nr. 3, 1995, Seiten 667-673, XP000979463 ISSN: 0888-7543 in der Anmeldung erwähnt
- WANG XIAODONG ET AL: "Cleavage of sterol regulatory element binding proteins (SREBPs) by CPP32 during apoptosis" THE EMBO JOURNAL, Bd. 15, Nr. 5, 1996, Seiten 1012-1020, XP002159140
- BROWN MS UND GOLDSTEIN JL: "The SREBP pathway: Regulation of cholesterol metabolism by proteolysis of a membrane-bound transcription factor" CELL,CELL PRESS, CAMBRIDGE, NA,US, Bd. 89, 2. Mai 1997 (1997-05-02), Seiten 331-340, XP002123067 ISSN: 0092-8674 in der Anmeldung erwähnt
- SHIMANO HITOSHI IICHIRO SHIMOMURA ET AL: "Elevated levels of SREBP-2 and cholesterol synthesis in livers of mice homozygous for a targeted disruption of the SREBP-1 gene." JOURNAL OF CLINICAL INVESTIGATION, Bd. 100, Nr. 8, 1997, Seiten 2115-2124, XP002931070 ISSN: 0021-9738
- HACIA JOSEPH G ET AL: "Strategies for mutational analysis of the large multiexon ATM gene using high-density oligonucleotide arrays." GENOME RESEARCH, Bd. 8, Nr. 12, Dezember 1998 (1998-12), Seiten 1245-1258, XP002925459 ISSN: 1088-9051

## Beschreibung

### Technisches Gebiet

Die vorliegende Erfindung betrifft Polymorphismen, insbesondere Desoxyribonukleinsäuren (DNA oder DNS)-Polymorphismen, in Sterol-Regulator Element-Bindenden Proteinen, insbesondere Protein-1 (SREBP-1) und Protein-2 (SREBP-2), resp. die Verwendung solcher Polymorphismen für die Diagnostik, aber auch für das WirkstoffScreening.

### Stand der Technik

Epidemiologische Langzeitstudien haben in den letzten Jahrzehnten zahlreiche Faktoren identifiziert, die die Atherosklerose-Entstehung beschleunigen, und damit die Entstehung von Herzinfarkten fördern. Trotz der Vermeidung von Umweltfaktoren und von Verhaltensweisen, die das Atherosklerose-Risiko erhöhen, kann es schon bei jüngeren Erwachsenen zu ausgeprägten atherosklerotischen Veränderungen bis hin zum Herzinfarkt kommen. In solchen Fällen spielen Erbfaktoren eine entscheidende Rolle. Es ist beispielsweise bekannt, dass Defekte von Genen, die im Cholesterinstoffwechsel eine wichtige Rolle spielen, die Medikation mit cholesterinsenkenden Mitteln erfordern. Durch die frühzeitige Erkennung eines genetischen Defekts lassen sich so rechtzeitig Gegenmassnahmen ergreifen.

Es ist deshalb sowohl vom diagnostischen als auch vom therapeutischen Standpunkt aus sehr wünschenswert wesentliche genetische Veränderungen erkennen und nachweisen zu können (siehe A. R. Miserez, Die Bedeutung genetischer Faktoren bei der Entstehung des Herzinfarkts, uni nova, April 1998, S. 44-52).

Cholesterin ist, neben seiner Eigenschaft als Vorläufer von Steroidhormonen und Gallensäuren, ein essentieller Bestandteil der Zellmembranen, der die Permeabilität der letzteren entscheidend beeinflusst. Menschliche Zellen kontrollieren ihren intrazellulären Cholesteringehalt in engen Grenzen durch die Regulation der rezeptorvermittelten Aufnahme extrazellulärer, cholesterinenthaltender Lipoproteinpartikel niedriger Dichte (low density lipoproteins, LDL) und durch die Steuerung der intrazellulären Cholesterinbiosynthese. LDL-Partikel binden sich an den LDL-Rezeptor (LDLR) mittels ihrer Apolipoprotein (Apo) B-Anteile. Die Bindung und die nachfolgende Internalisierung dieser Lipoprotein-Rezeptor-Komplexe kann teilweise oder vollständig unterbrochen sein, wenn eines der an diesem Prozess beteiligten Proteine defekt ist oder fehlt. Mutationen der Gene, die das Apolipoprotein E (Mutationen führen zu familiärer Dysbetalipoproteinämie (FDL)), das Apolipoprotein B-100 (Mutationen führen zu familiär-defektivem Apo B (FDB)), sowie den LDL-Rezeptor (Mutationen führen zu familiärer Hypercholesterinämie (FH)) kodieren, resultieren in einer Ansammlung von cholesterinenthaltenden Partikeln im Plasma, was mit einem Anstieg des Risikos für koronare Herzkrankheit einhergeht. Mit diese Mutationen können aber in den meisten untersuchten Populationen nur 4.2 bis 7 % der Fälle mit Hypercholesterinämie (definiert als die 10 % der Personen einer Population mit LDLC-Konzentrationen über dem 90ten Percentil) erklärt werden. Folglich sind die bei der Mehrzahl der betroffenen Personen mit erhöhtem Plasma-LDLC ursächlichen Gendefekten noch nicht identifiziert.

Die Promotoren des LDLR-Gens sowie der Gene, die in der Cholesterinbiosynthese eine Rolle spielen, wie zum Beispiel die Hydroxymethylglutaryl-(HMG)-CoA-Synthase-, die Farnesyl-Pyrophosphat-Synthase- und die Squalen-Synthase-Gene, enthalten spezifische Nukleotidsequenzen, die sogenannten Sterol-Regulator-Elemente (SRE).

Es ist ebenfalls bereits bekannt, dass zwei Proteine, die SRE-bindenden Proteine SREBP-1 und SREBP-2, an die SRE in den Promotoren dieser Gene binden und deren Transkriptionsrate aktivieren. Bei einem Mangel an Sterolen innerhalb der Zelle werden beide Proteine durch jeweils zwei proteolytische Schritte aktiviert, zunächst durch einen Sterol (bzw. Cholesterol)-sensitiven Schritt und danach durch einen Sterol-unabhängigen Schritt. Durch diese Proteolyseschritte entstehen 68 kDa grosse Proteine aus der NH₂ Region der sich im Zytoplasma befindlichen SREBP-1- und SREBP-2-Vorläuferproteine. Die am Amino-Ende freigesetzte, reife Form der Transkriptionsfaktoren wandert in den Zellkern und bindet sich an die SRE der Promotoren von Cholesterin-regulierenden Genen. In der Folge werden diese Gene aktiviert, was zu einer Erhöhung der rezeptorvermittelten Aufnahme von LDL und zu einer verstärkten intrazellulären Cholesterinbiosynthese führt.

Sobald sich Cholesterin in der Zelle anhäuft, wird der erste, Cholesterin-empfindliche Schritt inhibiert, die reifen Formen der SREBP verschwinden und die Transkriptionsraten vermindern sich, wodurch eine zu grosse Akkumulation von Cholesterin in der Zelle verhindert wird. SREBP-1 und SREBP-2 regulieren zahlreiche SREenthaltende Gene, die in die Cholesterin-Homöostase involviert sind, SREBP-1 aktiviert zusätzlich die HMG-CoA-Reduktase und die Squalen-Synthase. SREBP-1 und SREBP-2 sind Mitglieder der sogenannten basischen Helix-Loop-Helix Leucine Zipper Transkriptionsfaktor-Familie. Die Gene, die diese Faktoren kodieren, wurden kürzlich kloniert und in ihrer genetischen Struktur charakterisiert (20, 21).

Trotz all dieser bereits gewonnenen Erkenntnisse liegt aber - wie bereits oben erwähnt - die Zahl der erkennbaren Risikopatienten für z.B. Hypercholesterinämie bei unter 7 %.

Ziel der vorliegenden Erfindung war es deshalb, die Frühdiagnose und die Therapie von Risikopatienten zu verbessern.

Dieses Ziel wurde dadurch erreicht, dass Diagnoseverfahren sowie für deren Durchführung geeignete Polymorphismen auf den SREBP-Genen bereitgestellt werden, insbesondere Polymorphismen, die bei einem Teil der Patienten mit Änderungen im Lipidhaushalt, insbesondere im Cholesterinhaushalt, vorzugsweise bei einem bedeutenden Teil solcher Patienten, auftreten.

### Darstellung der Erfindung

Ein Gegenstand der vorliegenden Erfindung ist folglich ein Verfahren zur Erkennung eines erhöhten oder erniedrigten Krankheits- und/oder Mortalitätsrisikos und/oder einer erhöhten oder erniedrigten Sensitivität auf Therapieverfahren resp. deren Nebenwirkungen.

Weitere Gegenstände der vorliegenden Erfindung sind die Verwendung von Polymorphismen zur Diagnose, zur Beurteilung von Behandlungen gegen Krankheiten und zum Wirkstoffscreening, sowie die Bereitstellung geeigneter Polymorphismen.

Überraschenderweise wurde gefunden, dass Polymorphismen auf Sterol-Regulator Element-Bindenden-Proteinen (SREBP), insbesondere SREBP-1 und SREBP-2, indikatoren für Gesundheits- resp. Therapierisiken sind. Das erfindungsgemässe Verfahren ist deshalb dadurch gekennzeichnet, dass nach Blut- resp. Gewebeentnahme, das Blut resp. Gewebe auf das Vorhandensein eines Polymorphismus in mindestens einem SREBP untersucht wird, wobei das Vorhandensein eines Polymorphismus auf Amino- und/oder Nukleinsäure-Ebene bestimmt werden kann. Der Begriff Polymorphismus, wie er im Rahmen dieser Erfindung gebraucht wird, beschreibt jede natürlich im Menschen vorkommende Sequenzvariation, vorzugsweise aber eine Sequenzvariation, die bei einem grossen Bevölkerungsanteil vorkommt.

In einem bevorzugten Verfahren werden entsprechende Nukleinsäuresequenzen, die einen charakteristischen Polymorphismus aufweisen,insbesondere einen Polymorphismus von SREBP-1 und/oder SREBP-2, auf einem DNA- und/oder RNA-Chip verwendet, sog. Microarray (DNA Chip) Technologie. Andere Verfahren sind beispielsweise PCR und darauffolgende Restriktionsenzymverdauung, beispielsweise mit *Msp* I bzw. *Xmn* I; "Single Strand Conformation Polymorphism (SSCP)"-Verfahren; "Denaturing Gradient Gel Electrophoresis (DGGE)"-Verfahren; "Protein Truncation Test (PTT)"-Verfahren; "Restriction Fragment Length Polymorphisms (RFLP)"-Verfahren; "Cleavage Fragment Length Polymorphisms (CFLP)"-Verfahren; "Chemical Cleavage of Mismatches"-Verfahren; Sequenzierung; Minisequenzierung (Snap-shot-Sequenzierung); auf "High Pressure Liquid Chromatography (HPLC)" basierende Verfahren (dHPLC); auf Massenspektroskopie basierende Verfahren; Dot blot-Verfahren (allelspezifische Oligonukleotide); allelspezifisches PCR-Verfahren (allelspezifische Oligonukleotide); "Real-Time" quantitative PCR-Spectrophotometrie (z.B. *Taq*Man^{™}, Light Cycler^{™}) ; und "Luminescent non-gel based molcular interrogation".

Mit den im Rahmen dieser Erfindung speziell interessierenden Polymorphismen, insbesondere jenen, die auf den SREBP-1 und SREBP-2 Genen gefunden wurden, geht eine geänderte Funktionsfähigkeit der Proteine einher. In Anwesenheit der unten näher beschriebenen Mutationen im SREBP-1- und SREBP-2-Gen beispielsweise wird das LDL-Rezeptorgen weniger oder besser aktiviert, was zu veränderten Cholesterinspiegeln beim Menschen führt.

Ferner wurde im Rahmen der vorliegenden Erfindung gefunden, dass entsprechende Polymorphismen Indikatoren für erhöhtes oder erniedrigtes Krankheitsrisiko sind, insbesondere für erhöhtes resp. erniedrigtes Risiko an Hypercholesterinämie oder der Alzheimer-Krankheit zu erkranken. Sie ermöglichen auch die Beurteilung des Risikos für das Auftreten von Problemen bei der HIV-Therapie, insbesondere der Therapie mit Protasehemmern, und ermöglichen das Abschätzen des Risikos für die Entwicklung irgendeiner mit einem erhöhten Mortalitätsrisiko verbundenen Erkrankung, dies auch unabhängig von einer gegebenenfalls assoziierten Cholesterinmodifikation oder Alzheimer-Krankheit.

Die Erfindung wird unten und in den Figuren näher beschrieben.

### Kurze Beschreibung der Zeichnungen

Figur 1A zeigt ein Chromatogram zur Identifizierung des Exon-Polymorphismus in SREBP-1, und den ermittelten Polymorphismus, nämlich eine Mutation im SREBP-1 Gen (Exon 18c) an Aminosäureposition 1028 (G1028G), die zu keinem Aminosäureaustausch führt, aber eine *Xmn* I Restriktionsschnittstelle generiert.

Figur 1B zeigt ein Chromatogram zur Identifizierung des Exon-Polymorphismus in SREBP-2 und den ermittelten Polymorphismus, nämlich eine Mutation im SREBP-2 Gen (Exon 10) an Aminosäureposition 595 (A595G), die zu einer Aminosäuresubstitution führt (Alanin zu Glycin) und eine zusätzliche *Msp* I Restriktionsschnittstelle generiert.

Figur 2A zeigt, wie mittels PCR Amplifikation des ganzen Exon 18c (SREBP-1) und darauffolgende Restriktionsenzymverdauung homozygote und heterozygote Träger der entsprechenden Mutation beim Screenen von grossen Personenkollektiven mit hohem Durchsatz identifiziert werden können.

Figur 2B zeigt, wie mittels PCR Amplifikation des 5'-Endes von Exon 10 (SREBP-2) und darauffolgende Restriktionsenzymverdauung homozygote und heterozygote Träger der entsprechenden Mutation beim Screenen von grossen Personenkollektiven mit hohem Durchsatz identifiziert werden können.

Figur 3 zeigt für SREBP-1 und für SREBP-2 den Vergleich zwischen Trägern und Nicht-Trägern der Polymorphismen hinsichtlich der entsprechenden, gemittelten Gesamt-Cholesterinkonzentrationen und der Gen-Gen Interaktionen mit dem Apolipoprotein E-Gen.

Figur 4 zeigt die prozentuale Aenderung der Plasmacholesterinspiegel vor und nach Gabe von Proteasehemmern in Abhängigkeit vom G1028G Polymorphismus.

### Wege zur Ausführung der Erfindung

Polymorphismen in den SREBP-Genen allgemein können, wie unten für die SREBP-1- und SREBP-2-Gene beschrieben, ermittelt werden.

Die speziellen Polymorphismen können z.B. dadurch bestimmt werden, dass Oligonukleotide entworfen werden, die den Intron-Sequenzen der SREBP-1- und SREBP-2-Gene entsprechen, die unmittelbar den Exon-/Intron-Grenzen benachbart sind, und dass mittels der Einzel-Strang-Konformations-Polymorphismus-Methode (SSCP) beide Gene für Sequenzvariationen getestet werden.

Auf diese Weise wurden die folgenden relativ häufig auftretenden Polymorphismen gefunden, die jeweils dem normalen Gen gegenübergestellt werden, wobei NS die Nukleotidsequenz und AS die Aminosäuresequenz bedeuten:

| **SREBP-1 (Wild-Typ):** | | | | | | | | |
|---|---|---|---|---|---|---|---|---|
| NS | G | CAC | CTA | GGC | AAA | GGC | TTC | (Seq. Id. Nr. 1) |
| AS | | H | L | **G** | K | G | F | (Seq. Id. Nr. 2) |

| **SREBP-1-Exon 18c-Polymorphismus (SREBP-1-G1028G oder SREBP-1c-G1028G):** | | | | | | | | |
|---|---|---|---|---|---|---|---|---|
| NS | G | CAC | CTA | GG**G** | AAA | GGC | TTC | (Seq. Id. Nr. 3) |
| AS | | H | L | **G** | K | G | F | (Seq. Id. Nr. 4) |

| **SREBP-2 (Wild-Typ):** | | | | | | | | |
|---|---|---|---|---|---|---|---|---|
| NS | CT | GCT | GCC | GCC | AAC | CTA | CA | (Seq. Id. Nr. 5) |
| AS | A | A | A | **A** | N | L | Q | (Seq. Id. Nr. 6) |

| | | | | | | | | |
|---|---|---|---|---|---|---|---|---|
| **SREBP-2-Exon 10-Polymorphismus (SREBP-2-A595G):** | | | | | | | | |
| NS | CT | GCT | GCC | G**G**C | AAC | CTA | CA | (Seq. Id. Nr. 7) |
| AS | A | A | A | **G** | N | L | Q | (Seq. Id. Nr. 8) |

Analog wurde ein zwischenzeitlich im Rahmen einer Diplomarbeit veröffentlichter weiterer SREBP-2-Polymorphismus mit relativ grosser Häufigkeit ermittelt (siehe Diplomarbeit am Departement Forschung, Universitätskliniken Basel, von Patrick Y. Müller, "Sterol Regulatory Element Binding Protein 2: ..."), der in der Folge als SREBP-2-Exon-6-Polymorphismus oder SREBP-2-R371K bezeichnet wird.

Eine Gegenüberstellung der Sequenz des WildTyps und dieses weiteren Polymorphismus zeigt eine Änderung auf Proteinstufe, nämlich Mutation eines Arginins (R) zu einem Lysin (K) an Position 371 (R371K) in Exon 6, nämlich:

| **SREBP-2 (Wild-Typ):** | | | |
|---|---|---|---|
| NS | CTG | AGG | AAG |
| AS | L | R | K |

| **SREBP-2-Exon 6-Polymorphismus (SREBP-2-R371K):** | | | |
|---|---|---|---|
| NS | CTG | A**A**G | AAG |
| AS | L | K | K |

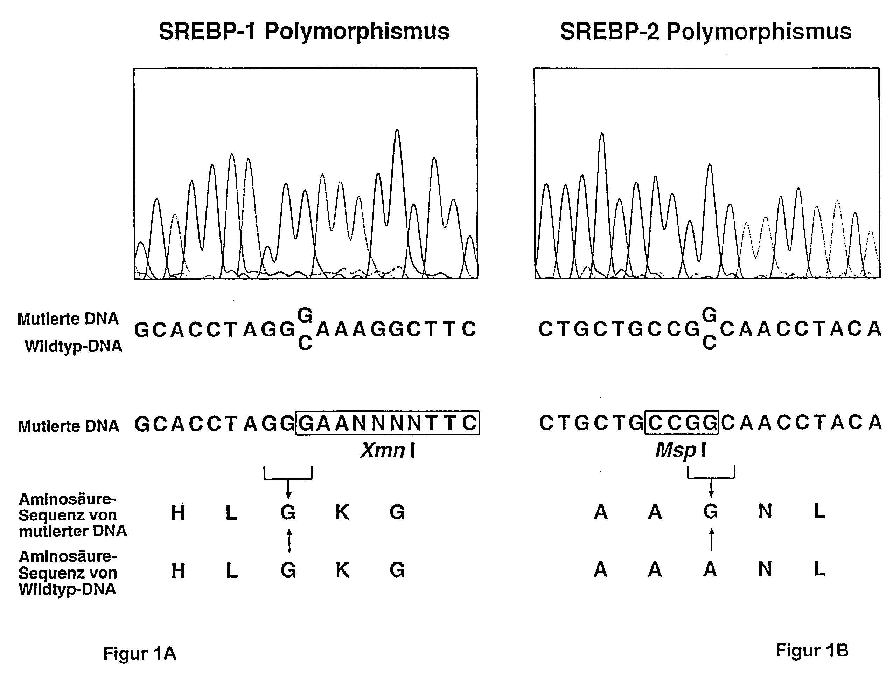

Wie aus den obigen Sequenzen ersichtlich ist, weist jeder der drei Polymorphismen eine geänderte Nukleinsäure im Exonbereich auf, wobei nur zwei der drei Polymorphismen, nämlich jene zu SREBP-2, eine Mutation aufweisen, die sich auf Proteinebene auswirkt (siehe dazu Figur 1A, Figur 1B). Alle drei Polymorphismen führen jedoch zu neuen Schnittstellen für Restriktionsenzyme, nämlich im SREBP-1 zu einer für *Xmn* I und im SREBP-2 zu einer für *Msp* I resp. einer für Dde I.

Selbstverständlich sind die entsprechenden Polymorphismen auch in den Komplementärsträngen vorhanden, so dass im Rahmen der vorliegenden Erfindung überall, wo Bezug auf Nukleotidsequenzen gemacht wird, die entsprechende Offenbarung die Komplementärsequenzen einschliesst.

Da der Polymorphismus im SREBP-1-Gen nicht zu einer Änderung auf Proteinebene führt aber trotzdem mit dem Auftreten von Hypercholesterinämie korrelierbar ist, drängt sich der Schluss auf, dass dieser Polymorphismus mit einer oder mehreren Mutationen im selben Gen einhergeht oder einen Einfluss auf der RNA-Ebene ausübt. Diese Annahme steht im Einklang mit der Tatsache, dass für den in SREBP-1 gefundenen Polymorphismus nicht nur ein Zusammenhang mit dem Auftreten von Hypercholesterinämie gefunden wird, sondern gleichzeitig ein Zusammenhang mit einem Mangel an Erhöhung der Gesamtcholesterin- und Triglycerid-Konzentration in HIV-Patienten nach der Verabreichung von Protease-Inhibitoren festgestellt wird. Dieser Polymorphismus ist deshalb ein wertvolles Hilfsmittel bei der Risikoabschätzung des Auftretens von unerwünschten Wirkungen und Einstellung einer Behandlung mit ProteaseHemmern.

Eine andere wichtige Eigenschaft der vorliegend beschriebenen Polymorphismen, insbesondere des SREBP-2-A595G-Polymorphismus, ist dessen Vorherrschen bei Patienten mit der Alzheimer-Krankheit im Vergleich zu dessen Vorkommen in der Bevölkerung allgemein, nämlich 7 % bei Alzheimer Patienten gegenüber 2.4 % in der Bevölkerung allgemein.

Überraschenderweise wurde zudem gefunden, dass alle drei oben näher beschriebenen Polymorphismen signifikanten Einfluss auf die Mortalität ihres Trägers haben.

Zusammenfassend kann somit festgehalten werden, dass SREBP-2-A595G speziell geeignet ist um Aussagen über das Risiko für Cholesterinerhöhung an und für sich zu machen, währenddem SREBP-1c-G1028G insbesondere geeignet ist als prognostischer Marker für die individuelle Reaktion (Risiko der Cholesterinerhöhung) nach Gabe von Medikamenten. Für Aussagen über das Risiko für die Entwicklung der Alzheimer-Krankheit (auch unabhängig von einer gegebenenfalls assoziierten Cholesterinerhöhung oder -erniedrigung) ist SREBP-2-A595G bevorzugt, während für die Bestimmung des Risikos für die Entwicklung einer Erkrankung, die mit einem erhöhten Mortalitätsrisiko verbunden ist (auch unabhängig von einer gegebenenfalls assoziierten Cholesterinmodifikation oder Alzheimer-Krankheit) alle drei oben näher beschriebenen Polymorphismen geeignet sind, wobei SREBP-2-A595G und SREBP-1c-G1028G bevorzugt sind.

Während allgemein für das Verfahren Polymorphismen der SREBP geeignet sind, sind solche von SREBP-1 und/oder SREBP-2 bevorzugt, insbesondere aber Polymorphismen, die zu einer erhöhten oder erniedrigten Aktivierung von Genen im Lipidstoffwechsel, insbesondere im Cholesterinstoffwechsel führen. Dabei sind Polymorphismen, die zu erhöhter oder erniedrigter Plasmakonzentration mindestens eines Lipids, insbesondere von Cholesterin, führen weiter bevorzugt.

Es hat sich gezeigt, dass ein Polymorphismus, der eine Erkennungssequenz für eine innerhalb des Polymorphismus liegende Schnittstelle aufweist,sich speziell für ein Verfahren unter Verwendung dieser Erkennungssequenz eignet. Solche Erkennungssequenzen sind beispielsweise die Erkennungssequenz für *Xmn* I oder *Msp* I, d.h. GAANNNNTTC oder CCGG, wobei N ein beliebiges Nukleotid sein kann. Sequenzen, die solche Erkennungssequenzen enthalten sind beispielsweise

SREBP-1, Exon 18c:GCACCTAGGGAAAGGCTTC, (Seq. Id. Nr. 3)und

SREBP-2, Exon 10: CTGCTGCCGGCAACCTACA (Seq. Id. Nr. 7).

Diese Sequenzen können als solche oder zusammen mit weiteren Nukleotiden aus ihrer natürlichen Nachbarschaft als z.B. Sonde eingesetzt werden. Daneben gibt es aber auch weitere geeignete Sequenzen, wie die folgende Nukleinsäure-Sequenz, gegebenenfalls zusammen mit weiteren Nukleotiden aus der natürlichen Nachbarschaft dieser Sequenz, nämlich

SREBP-2, Exon 6: CTGAAGAAG.

Ein bevorzugtes Verfahren auf Ebene der Nukleinsäuren ist dadurch gekennzeichnet, dass nach Blutresp. Gewebeentnahme und DNA-Extraktion mindestens ein Teilstück einer Sequenz, insbesondere eines Exons, eines SREBP, das einen Polymorphismus enthält, unter Verwendung zweier Oligonukleotidsequenzen amplifiziert wird, wobei der Polymorphismus charakteristisch ist für eine erhöhte oder erniedrigte Aktivierung von Genen im Lipidstoffwechsel insbesondere im Cholesterinstoffwechsel, und speziell bevorzugt für erhöhtes oder erniedrigtes Risiko für Hypercholesterinämie beim Menschen, und dass das Produkt der Amplifikation einer Verdauung mit geeigneten Restriktionsenzymen oder einer Denaturierung unterworfen wird und dass die Verdauungs- resp. Denaturierungsprodukte elektrophoretisch aufgetrennt werden.

Falls der Polymorphismus in einem Exon liegt ist vorzugsweise mindestens eine der Oligonukleotidsequenzenen im Intronbereich angesiedelt, der dem Exon benachbart ist, in dem der Polymorphismus existiert, wie beispielsweise die Paare
**S1.18cF** (Seq. Id. Nr. 9): 5'-TTATTTATAATCTGGGTTTTGTGTC-3' und
**S1.18cR** (Seq. Id. Nr. 10): 5'-GGGAAGAGCTAAGTTAAAAGTTGTG-3' oder
***Eco*R I.S1.18cF** (Seq.-Id. Nr. 11): 5'- CGGAATTCTGAAATTATTTATAATCTGGGTTTTGTGTC -3' und
***Eco*R I.*S*1.18cR** (Seq. Id. Nr. 12): 5'-CGGAATTCATCGGGGAAGAGCTAAGTTAAAAGTTGTG-3' oder
***S*2.10P.F** (Seq. Id. Nr. 13): 5'-GCCAGTGACCATTAACACCTTTTGA-3' und
***S*2.10P.R.** (Seq. Id. Nr. 14): 5'-TCGTCTTCAAAGCCTGCCTCAGTGGCTGGC-3' oder
***Eco*RI *S*2.10F** (Seq. Id. Nr. 15): 5'-CGGAATTCGCCAGTGACCATTAACACCTTTTGA-3' und
***Eco*RI *S*2.10R** (Seq. Id. Nr. 16): 5'-CGGAATTCTGCAGCAAGCCAGTCATCAGCAGCT-3'
***Eco*RI *S*2.6F** (Seq. Id. Nr. 17): 5'-CGGAATTCTGGTCTCACTGTGTTTTCACTCATC-3'
***Eco*RI *S*2.6R** (Seq. Id. Nr. 18): 5'-CGGAATTCGCCAGGGCTGACAAGCCTTTTCTCA-3'.

Neben den oben angegebenen Sequenzen resp. Sequenzpaaren sind auch andere Sequenzen resp. Sequenzpaare verwendbar, wie mit den oben angegebenen Sequenzen unter stringenten Bedingungen hybridisierbare Sequenzen, einschlisslich Sequenzen ohne resp. mit anderen Erkennungssequenzen als der oben angegebenen EcoRI-Sequenz. Die Gesamtlänge solcher Sequenzen beträgt üblicherweise 15 bis 30 Basen.

Geeignete Polymorphismen können ermittelt werden durch amplifizieren und analysieren einer interessierenden SREBP-Sequenz, Vergleich der Exon-Bereiche dieser interessierendne Sequenz mit den Exon-Bereichen der Sequenz des in einer Population am häufigsten auftretenden Typs des entsprechenden SREBP und Untersuchung der Sequenzen mit gefundenen Unterschieden auf Fehlfunktion, wobei vorzugsweise die Unterschiede zu einer anderen Aminosäure und/oder insbesondere zu einer Erkennungsstelle für ein Restriktionsenzym führen. Eine solche Erkennungsstelle liegt vorzugsweise in einem Exon, sie kann aber auch in einem Intron liegen und beispielsweise zu einer Spleissvariante führen.

Der grosse Einfluss der gefundenen Polymorphismen auf Krankheitsbilder beeinflussende Faktoren wird in der Folge kurz anhand der häufiger auftretenden Polymorphismen A595G und G1028G diskutiert: Die A595G Mutation im SREBP-2 Gen ist mit einer signifikanten Modifikation der gemittelten Plasmacholesterin-Konzentrationen assoziiert. Die der publizierten cDNA Sequenz(12,15,16) entsprechende Aminosäurefolge wurde als Wildtyp definiert, obgleich - zumindest in dem vorliegend untersuchten Schweizer Bevölkerungskollektiv - die Sequenz, die Glycin an Position 595 kodiert, eine viel höhere Prävalenz hatte als das publizierte Alanin an dieser Stelle. Ueber 93% aller Individuen waren heterozygote oder homozygote Träger der A595G Mutation. Beide Gene wurden aus einer cDNA-Bibliothek, abgeleitet von HeLa Zellen, die vom Karzinom einer afro-amerikanischen Frau (Henriette Lacks) abstammen, sequenziert(17). Direkte Versuche mit HeLa Zellen zeigten Homozygotie hinsichtlich des nicht mutierten A595A Genotyps und legten die Annahme nahe, dass diese Person homozygote Trägerin des Wildtyp Allels war - ein Zustand, der jedoch nur bei 6.69% des schweizerischen Bevölkerungskollektivs vorgefunden wurde. Die Beobachtung einer hohen Prävalenz der A595G Mutation, führte im Rahmen der vorliegenden Erfindung zur Annahme, dass der seltene Wildtyp in homozygoter Form (11) mit einer höheren Plasmacholesterinkonzentration assoziiert sei, und die nicht mutierte Form (22) mit einer niedrigeren Konzentration, dass somit ein autosomal-rezessiver Effekt vorliegen könnte, weshalb die Allelkombinationen 11 und 12/22 miteinander verglichen wurden.

Das Kollektiv der eingeschlossenen Individuen war heterogen hinsichtlich der Plasmagesamtcholesterinkonzentrationen, die von 1.95 bis 22.65 mmol/L reichten. Dieser grosse Bereich erklärte sich durch den Einschluss von zufälligen Stichproben, aber auch ausgewählten Kollektiven, und folglich normocholesterinämischen und hypercholesterinämischen Individuen. Daher war es nicht verwunderlich, dass ohne Stratifizierung des Kollektivs in zufällig/nicht zufällig ausgewählte oder in normocholesterinämsche/hypercholesterinämische Untergruppen, der Effekt zumindest eines der Polymorphismen, der des G1028G Polymorphismus, keine statistische Signifikanz erreichte (P=0.0770). Sobald jedoch diese Auswahlkriterien in die Berechnungen einbezogen wurden, war der Unterschied der G1028G Allelkombinationen 11/12 gegenüber 22 signifikant (P=0.0164). Ebenso verringerte sich bei der A595G Mutation die Wahrscheinlichkeit, dass Unterschiede in den Plasmagesamtcholesterinkonzentrationen zwischen der Allelkombination 11 gegenüber 12/22 auf Zufall beruhten, von P=0.0003 (ungepaarter t-Test, keine Stratifizierung) auf P<0.0001 (Varianzanalyse = ANOVA, Stratifizierung.

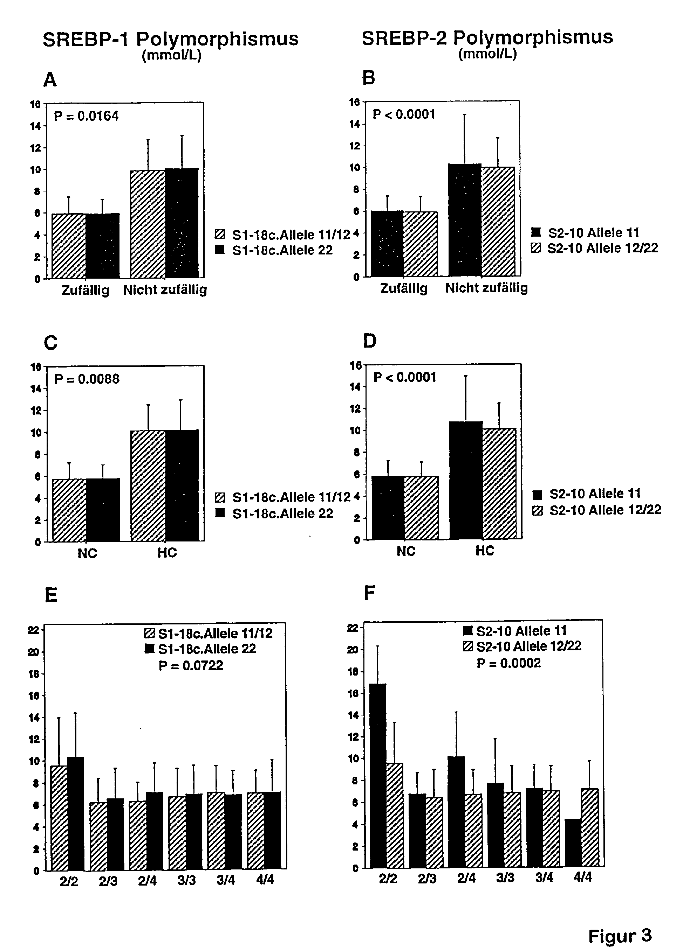

Darüberhinaus waren bei beiden Polymorphismen die Assoziationen zwischen einer bestimmten Allelkombination und höheren Plasmacholesterin-Konzentrationen (Allelkombination 22 beim G1028G Polymorphismus, Allelkombination 11 beim A595G Polymorphismus; in Figur 3, E und F, schwarz dargestellt) in Anwesenheit des R158C (ε2 Phänotyp) Polymorphismus stärker und in Anwesenheit des C112R (ε4 Phänotyp) Polymorphismus im Apo E Gen schwächer. Es wurde gefunden,dass diese Gen-Gen Interaktionen die Assoziation des G1028G Polymorphismus (22 Allelkombination) mit höheren Plasmacholesterinkonzentrationen deutlich beeinflussen: nach Ausschluss der Träger der C112R Mutation war der Effekt der G1028G C → G Mutation in homozygoter Form (22) hoch signifikant (P=0.0002).

Es konnte somit gezeigt werden, dass beide SREBP-Gene die Plasmacholesterin-Konzentrationen beim Menschen ähnlich den bekannten Effekten der beiden Polymorphismen im Apo E Gen (C112R und R158C), modifizieren. Desweiteren wurden Gen-Gen Interaktionen deutlich, wenn die SREBP-1 und -2 Genpolymorphismen mit den Polymorphismen im Apo E Gen korreliert wurden.

Bei 11.6% der Individuen mit sekundären Hyperlipoproteinämien waren die Plasma-triglyceridkonzentrationen erhöht. Ein Effekt erhöhter Triglyceridkonzentrationen wird durch die Tatsache unterstrichen, dass, wurden Individuen mit erhöhten Triglyceridkonzentrationen ausgeschlossen, die A595G Mutation eine signifikante Auswirkung auf männliche Individuen mit Diabetes mellitus zeigte (P=0.0018), dieser Effekt jedoch ausblieb, wenn Individuen mit erhöhten Triglyceridkonzentrationen eingeschlossen wurden.

Die A595G Mutation im SREBP-2 Gen könnte sowohl eng mit einer anderen Mutation verbunden sein als auch die Spaltungsrate des Proteins direkt betreffen. Exon 10, wo die A595G Mutation lokalisiert ist, gehört zwar nicht zum Teil des reifen Proteins, das in den Zellkern wandert. Trotzdem ist dieser Teil des Proteins insofern mit der Aktivität des Proteins verbunden, als er die Spaltungsvorgänge, die die SREBP-2 Vorläuferform aktivieren, beeinflusst.

Die Proteolyse wird durch ein Enzym eingeleitet, das eine hoch konservierte RXXL Sequenz der SREBP Vorläuferformen erkennt, welche in der hydrophilen Schleife lokalisiert ist. Durch einen ersten Proteolyseschritt werden die NH₂-Terminus- und die COOH-Terminus-Domänen getrennt. Nach diesem ersten, Sterol-sensitiven Schritt wird das verbleibende, Membran-gebundene NH₂-Terminus Fragment durch einen zweiten, Sterol-unabhängigen Schritt freigesetzt. Der zweite Proteolyseschnitt (site-2) ist innerhalb der Membran-durchspannenden Region lokalisiert und wird durch das site-2 Enzym vermittelt. Der zweite Schritt erfolgt nur, wenn die site-1 Proteolyse stattgefunden hat. Eine Vorbedingung für die site-1 Proteolyse ist jedoch die Bildung eines Komplexes aus SREBP und dem sogenannten SREBP Cleavage Activating Protein (SCAP). Bei Mangel von Sterolen in der Zelle bindet dieses Protein an die COOH-Terminus Domäne. Die Bildung des SREBP-SCAP Komplexes ist entscheidend für den site-1 Proteolyseschritt und abhängig von der Vollständigkeit der COOH-Terminus Domänen von SREBP-2 und SCAP (18,19). Auf der Grundlage der vor kurzem von Sakai et al. (18) durchgeführten Experimente, der den COOH-Terminus Teil der SREBP Vorläuferformen als regulatorische Einheit identifizierte, wirft die Mutation in dieser Domäne, die ein signifikantes Sinken der gemittelten Plasmacholesterin-Konzentrationen verursacht, die Frage einer leicht vereinfachten Bildung des SREBP-SCAP Komplexes auf, wenn die A595G Mutation vorhanden ist.

Der Effekt des G1028G Polymorphismus scheint von den Gen-Gen Interaktionen mit dem Apo E Gen beeinflusst zu sein. Im Gegensatz zur G1028G Mutation im SREBP-1 Gen, hat die A595G Mutation im SREBP-2 Gen als Marker signifikante Auswirkungen auf die Plasmacholesterin-Konzentrationen sowohl wenn das gesamte Kollektiv untersucht wird, als auch bei der Analyse der verschiedenen Untergruppen. Es ist wahrscheinlich, dass die Mutation ihre Wirkung zeigt, indem sie direkt den Spaltungsvorgang beeinflusst, der für die Sterol-abhängige Aktivierung von SREBP-2 verantwortlich ist.

Aus den obigen Ausführungen folgt, dass beide Gene die individuellen Plasmacholesterin-Konzentrationen signifikant modifizieren. Obgleich viele Gene im intra- und extrazellulären Cholesterinstoffwechsel eine Rolle spielen, ist im Hinblick auf die Allgemeinbevölkerung bislang nur das Apo E Gen als modifizierendes Gen von grösserem Nutzen gewesen. Andere am Lipoproteinmetabolismus beteiligte Gene, wie etwa das LDL Rezeptorgen, das Apo B-100 Gen oder ein weiteres, unbekanntes Gen auf Chromosom 1p34.1-p32 könnten im mutierten Zustand deutliche Auswirkungen auf die Plasmagesamtcholesterinkonzentrationen haben. Jedoch sind diese Mutationen im Vergleich zu den Polymorphismen im Apo E- und den jetzt entdeckten Polymorphismen im SREBP-1 und SREBP-2 - Gen sehr selten. Selbst die R3'500Q Mutation, die mit einem von 209 betroffenen Individuen der Allgemeinbevölkerung (in der Schweiz) die höchste bisher beobachtete Prävalenz hat (2), tritt nicht häufig genug auf, um an diesem Polymorphismus den Einfluss eines bestimmten Gens auf den Cholesterin-stoffwechsel der Allgemeinbevölkerung zu untersuchen.

Die Verfahren und Polymorphismen der vorliegenden Erfindung sind somit sehr wertvolle Hilfsmittel für die Früherkennung von Risikopatienten sowie für die Optimierung von Prophylaxe und Therapien. Ferner eignen sie sich als Targets für das Wirkstoffscreening, sowie die Beurteilung einer Therapie gegen eine Krankheit wie z.B. HIV. Der Wert der bevorzugten Polymorphismen dieser Erfindung ist auch die Anwesenheit von Erkennungssequenzen in nächster Nähe des Polymorphismus. Diese Erkennungssequenzen sind in SREBP-1 die Erkennungssequenz für *Xmn* I , nämlich GAANNNNTTC, , wobei N ein beliebiges Nukleotid sein kann, und in SREBP-2 die Erkennungssequenz für *Msp* I, d.h. CCGG.

Die vorliegende Erfindung betrifft auch ein Verfahren zur Erfassung von Risikoträgern sowie Hilfsmittel für dieses Verfahren, wie Oligonukleotid-Sequenzen zur Amplifizierung interessierender DNA-Abschnitte.

Ein bevorzugtes Verfahren zur Erfassung von Risikoträgern zeichnet sich aus durch die nachfolgend aufgeführten Schritte:
1. Blut- oder Gewebeentnahme
2. DNA-Extraktion
3. Amplifikation mit geeignetem Primer
4. Verdauung mit geeigneten Restriktionsenzymen oder Denaturierung des PCR-Produktes
5. Elektrophoretische Auftrennung auf geeignetem Gel.

Über die Verdauung mit geeigneten Restriktionsenzymen werden insbesondere die speziell interessierenden Poymorphismen nachgewiesen und über die Denaturierung (Einzel-Strang-Konformations-Polymorphismus = SSCP) lassen sich daneben weitere Mutationen auffinden.

Eine bevorzugte Oligonukleotid-Sequenz zur Amplifizierung eines DNA-Abschnitts, der einem Exon-Bereich entspricht, in dem ein Polymorphismus existiert, ist dadurch gekennzeichnet, dass sie in einem Intronbereich angesiedelt ist, der dem Exon benachbart ist, in dem der Polymorphismus existiert und nahe der Exon/Intron Grenze liegt, oder im Exon selbst, sofern sich dadurch die Zahl der Schnittstellen vermindern lässt.

Bevorzugte Oligonukleotide für den SREBP-1 Polymorphismus sind die Oligonukleotide S1.18cF (Seq. Id. Nr. 9): 5'-TTATTTATAATCTGGGTTTTGTGTC-3' und S1.18cR (Seq. Id. Nr. 10): 5'-GGGAAGAGCTAAGTTAAAAGTTGTG-3', die auch die Erfassung von Spleiss-Mutationen gestatten, sowie Oligonukleotiden, die daneben zusätzliche *EcoR* I-Schnittstellen besitzen, wie EcoR I.S1.18cF (Seq. Id. Nr. 11): 5'- CGGAATTCTGAAATTATTTATAATCTGGGTTTTGTGTC -3' und *EcoR* I.S1.18cR (Seq. Id. Nr. 12): 5'-CGGAATTCATCGGGGAAGAGCTAAGTTAAAAGTTGTG-3'. Um Exon 10 des SREBP-2-Gens inklusive dessen Exon-/Intron-Grenzen zu amplifizieren sind die Oligonukleotide S2.10P.F (Seq. Id. Nr. 13):5'-GCCAGTGACCATTAACACCTTTTGA-3' und S2.10P.R (Seq. Id. Nr. 14):5'-TCGTCTTCAAAGCCTGCCTCAGTGGCTGGC-3' resp. *Eco*RI S2.10F (Seq. Id. Nr. 15): 5'-CGGAATTCGCCAGTGACCATTAACACCTTTTGA-3' und EcoRI S2.10R (Seq. Id. Nr. 16): 5'-CGGAATTCTGCAGCAAGCCAGTCATCAGCAGCT-3' bevorzugt.

Eine spezielle Verwendung von SREBP- Polymorphismen betrifft deren Einsatz auf sogenannten DNA- oder Gen-Chips. Verfahren unter Verwendung solcher Chips, die die gleichzeitige Erkennung verschiedener Gendefekte ermöglichen, sind in der Literatur beschrieben. So existiert Informationsmaterial der Firma Affymetrix zu deren GeneChip^{™} -Systemen, aber auch Artikel in Fachzeitschriften wie jene von Mark Chee et al., Accessing Genetic Information with High-Density DNA Arrays, Science Vol. 274, Seiten 610-4, (1996) und David G. Wang, Large-Scale Identification, Mapping, and Genotyping of Single-Nucleotide Polymorphisms in the Human Genome, Science Vol. 280, Seiten 1077-82, (1998).

Das Verfahren lässt sich kurz wie folgt zusammenfassen: Mittels Photolithographie werden gezielte Bereiche eines Wafers Schritt für Schritt der chemischen DNA- oder RNA-Einzelstrang-Synthese zugeführt, wobei der Schutzfilm nach jedem Syntheseschritt neu angebracht und anschliessend selektiv nur an denjenigen Stellen entfernt wird, an denen ein bestimmtes Nukleotid eingeführt werden soll. Durch dieses Vorgehen lassen sich Bereiche herstellen, die selektiv für bestimmte Polymorphismen sind. Zur Sichtbarmachung von Hybridisierungen mit Zielsequenzen können übliche Markierungen verwendet werden, z.B. lichtemittierende Markierung der in der Probe vorhandenen Fragmente, wie z.B. Biotinylierung und Detektion mit Streptavidin oder auch Fluoreszenzmarkierung.

Durch Entfernen markierter, nicht hybridisierender Fragmente werden Hybridisierungen auf dem Chip direkt oder nach einer weiteren Behandlung sichtbar. Selbstverständlich können die einzelnen Schritte dieses Verfahrens variiert werden, z.B. was den Zeitpunkt der Markierung oder die Art der Markierung anbelangt. Solche Variationen sind für den Fachmann erkennbar.

Ein Chip, der für die Früherkennung von Patienten mit einem erhöhten Risiko an Hypercholesterinämie geeignet ist weist neben den normalen SREBP-1- und SREBP-2-Sequenzen die entsprechenden Polymorphismen auf, die Gegenstand der vorliegenden Erfindung sind. Neben diesen SREBP-1- und SREBP-2-Analysemitteln können selbstverständlich auch entsprechende Sequenzen für die weiteren Polymorphismen vorhanden sein, die für Hypercholesterinämie charakteristisch sind, d.h. FH, FDB und FDL.

Selbstverständlich kann ein entsprechender Chip auch zur Diagnose anderer Krankheiten bestimmt werden, die eine Abhängigkeit von SREBP-1 und/oder -2 zeigen, wie Alzheimer-Krankheit, oder er kann zur gleichzeitigen Diagnose mehrerer Krankheiten oder Risikofaktoren gestaltet werden, indem darauf für die interessierenden Krankheiten oder Risiken charakteristische Polymorphismen angebracht werden. Diesbezüglich interessante Sequenzen sind beispielsweise für das Studium kardiovaskulärer Risiken Sequenzen aus der folgenden Gruppe (bevorzugte Sequenzen unterstrichen):

11β-Hydroxylase Aldosteron-Synthase Gen, 11β-Hydroxysteroid-Dehydrogenase (HSD11K)Gen, 17α-Hydroxylase (CYP17A)Gen, 3-Hydroxy-3-Methylglutaryl (HMG) Coenzym A Reduktase Gen, 3-Hydroxy-3-Methylglutaryl (HMG) Coenzym A Synthase Gen, Acyl Coenzym A:diacylglycerol acyltransferase Gen, Acyl-Coenzym A:cholesterol acyltransferase (ACAT)-1 Gen, Alpha-1-Antichymotrypsin Gen, Alpha-1-trypsin Gen, Alpha-Glaktosidase A Gen, Alpha-L-Iduronidase (IDUA) Gen, Alpha-Lecithin cholesterol acyltransferase (LCAT) Gen, Alpha-Synuclein Gen, Angiotensin Gen, Angiotensin II Typ 1 Rezeptor Gen, Angiotensin-converting Enzym Gen, Antitrypsin Gen, Apolipoprotein (a) Gen, Apolipoprotein AI-CIII-AIV Gen cluster, Apolipoprotein B-100 Gen, Apolipoprotein CI Gen, Apolipoprotein E (epsilon 2), Apolipoprotein E (epsilon 4), Apolipoprotein E Gen, Apolipoprotein E Rezeptor 2 Gen, Benzodiazepine Rezeptor Gen, CD-36 Gen, Cholesterol 24-Hydroxylase Gen, Cholesteryl ester transfer Protein (CETP) Gen, Cystathionin-β-Synthase Gen, Cystatin C Gen, Cytochrome P450 cholesterol side-chain cleavage Enzym Gen, Epithelialer Na⁺-Kanal (β-Untereinheit) Gen, Farnesyl-Pyrophosphate (PP) Synthase Gen, Fibrinogen Gen, Glucokinase Gen, GLUT1 Glukose Transporter Gen, Hepatische Lipase Gen, High density lipoprotein (HDL) Rezeptor Gen, Homogentisinsäure-Oxidase Gen, Hormone-sensitive Lipase Gen, Iduronat-2-Sulfatase Gen, Interleukin-8 Gen, Lecithin cholesterol acyltransferase (LCAT) Gen, Lipooxygenase Gen, Lipoprotein Lipase Gen, Low density lipoprotein receptor-related Protein (LRP) Gen, Low density lipoprotein Rezeptor Gen, Lysosomale saure Lipase Gen, Makrophagen Scavenger Rezeptor (SR-A) Gen, Makrophagen Scavenger Rezeptor (SR-BI) Gen, Methylene-tetrahydrofolate Reduktase Gen, Microsomal triglyceride transfer Protein (MTP) Gen, NF-_{κ}B Gen, Niemann-Pick C1 Protein Gen, Oxysterol binding Protein (OSBP) Gen, Paraoxonase-1 Gen, Paraoxonase-2 Gen, Peroxisome proliferator-activated receptor (PPAR) alpha Gen, Peroxisome proliferator-activated receptor (PPAR) beta Gen, Peroxisome proliferator-activated receptor (PPAR) gamma Gen, Plasminogen activator-inhibitor-1 Gen, Site-1 Protein (S1P) Gen, Site-2 Protein (S2P) Gen, Squalene Synthase Gen, SREBP cleavage-activating Protein (SCAP) Gen, Steroid acute regulatory Protein (StAR) Gen, Steroid-11β-Hydroxylase (CYP11B1) Gen, Sterol-27-Hydroxylase Gen, Sterol regulatory element-binding protein (SREBP)-1a Gen, Sterol regulatory element-binding protein (SREBP)-1c Gen, Sterol regulatory element-binding protein (SREBP)-2 Gen, Very low density lipoprotein (VLDL) Rezeptor Gen.

Für das Studium neurologischer Risiken ist beispielsweise ein Chip umfassend Sequenzen aus der nachfolgend aufgeführten Gruppe günstig (bevorzugte Sequenzen unterstrichen):

A-beta precursor Gen, Adenosin monophosphate deaminase Gen, Alpha 2-monoglobulin Gen, Alpha-1-Antichymotrypsin Gen, Alpha-1-trypsin Gen, Alpha-2 Macroglobulin Gen, Alpha-ketoglyterate dehydrogenase Gen, Amyloid betaprotein precursor Gen, Amyloid precursor Protein Gen, Amyloid precursor-like Protein 1 Gen, Amyloid precursor-like Protein 2 Gen, Antitrypsin Gen, Apolipoprotein (a) Gen, Apoliprotein AI-CIII-AIV Gen cluster, Apolipoprotein E (epsilon 2), Apolipoprotein E (epsilon 4), Apolipoprotein E Gen, Apolipoprotein E Rezeptor 2 Gen, Bcl-2 Gen, Beta-amyloid precursor Protein Gen, Beta-nerve growth factor Gen, Calbindin-D Gen, Captase Gen, Cathepsin D Gen, CD36 Gen, Clusterin Gen, Cyclooxygenase-2 Gen, Cystatin C Gen, Cytochrome C Oxidase 1 Gen, Cytochrome C Oxidase 2 Gen, Cytochrome Oxidase Gen, Dihydrofolate Reduktase Gen, Dihydrolipoylsuccinyltransferase (DLST) Gen, Endopeptidase 1 Gen, Estrogen-Bcl xL Gen, Fe65L2 Gen, Gamma-synuclein Gen, Gelsolin Gen, GLUT1 Glukose Transporter Gen, GLUT4 Glukose Transporter Gen, Glutaminsäure Decarboxylase Gen, Glutation S-transferase Gen, HLA-A2 Gen, Interleukin-1 Gen, Interleukin-6 Gen, Interleukin-8 Gen, L-3-Hydroxyacyl-Coenzym A Dehydrogenase Gen, Lipooxygenase Gen, Low density lipoprotein receptor-related Protein (LRP) Gen, Low density lipoprotein Rezeptor Gen, Makrophagen Scavenger Rezeptor (SR-A) Gen, Makrophagen Scavenger Rezeptor (SR-BI) Gen, Methylene-tetrahydrofolate Reduktase Gen, Myeloperoxidase Gen, NF-κB Gen, Niemann-Pick C1 Protein Gen, Non-A-beta component for amyloid (NAC) peptide Gen, Notch Gen, Ornithine transcarbamylase Gen, Presenilin 1 Gen, Presenilin 2 Gen, Prion Protein Gen (PRNP) Gen, Prostaglandin E2 Gen, Serotonin Gen, Serotonin Transporter Gen, Site-1 Protein (S1P) Gen, Site-2 Protein (S2P) Gen, SREBP cleavage-activating Protein (SCAP) Gen, Sterol regulatory element-binding protein (SREBP)-1a Gen, Sterol regulatory element-binding protein (SREBP)-1c Gen, Sterol regulatory element-binding protein (SREBP)-2 Gen, Superoxid dismutase gene Gen, Tau (Protein) Gen, Very low density lipoprotein (VLDL) Rezeptor Gen, X11alpha Protein Gen, X11L2 Gen.

Ferner sind die Polymorphismen, Verfahren und Chips der vorliegenden Erfindung auch geeignet um alfällige Risikopatienten für die Behandlung mit speziellen Medikamenten zu ermitteln, wie eine Behandlung mit Protease-Hemmern bei HIV-Infizierten.

Die vorliegende Erfindung wird nun anhand von Beispielen näher erläutert. Die Erfindung ist aber nicht auf die im experimentellen Teil beschriebenen Beispiele, resp. die darin explizit genannten Ausführungsformen, beschränkt.

### Experimenteller Teil

### Vorbemerkungen

Die Polymorphismen wurden ermittelt, indem Oligonukleotide zu Intronsequenzen im Exon/Intron-Grenzbereich synthetisiert wurden, so dass auch allfällige Spleissvarianten erfasst werden konnten. In der Folge wird das genaue Vorgehen bei der Detektion der vorliegend relevanten Polymorphismen sowie deren Untersuchung im Detail beschrieben.

### Probanden

Es wurden insgesamt 3'078 Personen in die Studie aufgenommen. DNA Polymorphismen und seltene Mutationen in fünf verschiedenen Genen wurden untersucht. In allen Personengruppen wurden Individuen mit TC Plasmakonzentrationen unter der 90. Percentile, standardisiert für Alter und Geschlecht, als normocholesterinämisch (NC) bezeichnet; Individuen mit TC Plasmakonzentrationen über der 90. Percentile als hypercholesterinämisch (HC). 1'685 Probanden aus verschiedenen prospektiv untersuchten Zufallsstichproben wurden eingeschlossen. 630 Individuen waren aus der "Swiss PREvalence for Apolipoprotein Defects" (SPREAD) Studie, einer grossen Querschnittsuntersuchung, die unverwandte, männliche Individuen aus den deutsch-, französisch- und romanischsprachigen Teilen der Schweiz einschloss, die für den Militärdienst rekrutiert wurden. Weitere 324 Individuen wurden von der Interdisziplinären Altersstudie (IDA) eingeschlossen. Weitere 413 ältere Individuen wurden eingeschlossen, die aufgrund einer vermuteten Beeinträchtigung der Gedächtnisfunktion untersucht wurden, aber nicht aufgrund einer Hypercholesterinämie. Diese Individuen aus der Basler Memory Clinic (BMC) wurden als weiteres Kontrollkollektiv in die Studie aufgenommen. 318 betroffene und/oder nicht betroffene Individuen wurden von der "Study to Investigate the Molecular Basis of Hypercholesterolemia in Switzerland in Hyperlipidemic Individuals by Pedigree Analysis" (SIBSHIP), einer Unterstudie des schweizerischen MED PED (Make Early Diagnosis - Prevent Early Death)-Programms, eine multinationalen Studie unter der Schirmherrschaft der WHO, in die Studie aufgenommen. 871 Individuen wurden von Kollektiven mit vermuteten primären und sekundären Hyperlipoproteinämien eingeschlossen. Die molekulare Diagnose beruhte auf der Identifikation der zugrundeliegenden Mutation (familiär-defektives Apo B (FDB), familiäre Dysbetalipoproteinämie (FDL), familiäre Hypercholesterinämie, molekular diagnostiziert (FHM) oder mittels Kosegregations-Analyse (FHM)). Die klinische Diagnose von familiären Formen der Hypercholesterinämie beruhte auf Gesamt- und/oder LDL-Cholesterinwerten oberhalb der 90. Percentile und einer Familienanamnese mit mindestens zwei weiteren Familienmitgliedern mit Hypercholesterinämie. Individuen mit Familien mit diesen Merkmalen und Triglyzeridwerten < 3.7 mmol/L und/oder Sehnenxanthomen wurden als familiäre Hypercholesterinämie, klinisch diagnostiziert, klassifiziert (FHC). Familien mit Individuen ohne Xanthome sowie Triglyzeridwerten ≥ 3.7 mmol/L wurden als familiär-kombinierte Hyperlipidämie (FCH) bezeichnet. Insgesamt 298 Personen stammten aus der "Study on the molecular basis of Triggers Activating a Rise in Triglycerides and cholesterol in Endocrinological and Renal Diseases" (STARTER). Ein Kollektiv von 130 Personen mit biochemisch bestätigtem Diabetes mellitus (Nüchtern-Plasma-Glukosewerte > 7.8 mmol/L) (DIA), 78 Individuen mit Hypothyreose und 14 Individuen mit Niereninsuffizienz (Kreatinin Clearance < 50ml/min) (RIN) wurden in die Studie eingeschlossen. Bei allen Personen wurden mindestens Alter, Geschlecht und Gesamtcholesterinkonzentrationen ohne lipidsenkende Behandlung und die klinische bzw. molekularbiologische Diagnose erfasst. Mit Ausnahme der SPREAD Studie wurden die Personen zusätzlich ausführlich klinisch charakterisiert. In der IDA, BMC, SIBSHIP und STARTER Studie wurden Grösse, Gewicht, Body-Mass-Index, Blutdruck, das Vorhandensein oder Fehlen von klinischen Anzeichen der Hypercholesterinämie (Sehnenxanthome, Xanthelasmen und Arcus lipoides) und Zeichen und Symptome von koronarer Herzkrankheit, zerebrovaskulären Erkrankungen und peripher-arterieller Verschlusskrankheit, sowie biochemische Parameter wie Plasmakonzentrationen von Gesamtcholesterin, LDL-Cholesterin, HDL-Cholesterin, Triglyceride und Schilddrüsen-stimulierendes Hormon (TSH) bestimmt. Die Dokumentation beinhaltete auch die persönliche Anamnese einer koronaren Herzkrankheit, einer zerebrovaskulären Erkrankung, einer peripher-arteriellen Verschlusskrankheit, einer Schilddrüsenerkrankung, eines Diabetes mellitus, das Ausmass von Alkohol- und Zigarettenkonsum (in pack years) und, in den SIBSHIP- und STARTER-Studien, eine ausführliche Familiengeschichte mit zusätzlichen Lipoproteinanalysen (z.B. Lipoprotein (a) [Lp(a)], Apolipoprotein B etc.). Bei allen diesen Personen wurden die Proben für die weiteren Tests anonymisiert.

### Material

Es wurden *Thermus aquaticus* DNA Polymerase und Desoxynukleotide von Perkin Elmer Cetus Corporation (Norwalk, CT, USA) und von Qiagen (Milden, Deutschland) verwendet. Die Restriktionsendonukleasen waren von New England Biolabs Inc. (Beverly, MA, USA) und vorgefärbte Protein-Molekulargewichts-Marker und DNA-Molekulargewichts-Marker von Roche Diagnostics (Basel, Schweiz). Die verwendeten Oligonukleotide wurden durch die Microsynth Inc. (Balgach, Schweiz) synthetisiert. Die DNA wurde in 200 µl Reaktionsgefässen mittels PCR Maschinen von Perkin Elmer (GeneAmp^{®} PCR-System 9700) und Stratagene (RoboCycler^{®} Gradient 96 temperature cycler, Stratagene, La Jolla, CA, USA) amplifiziert. Es wurde Agarose von BioRad (Irvines, CA, USA) und Polyacrylamid (Acrylamid: Bisacrylamid 37.5:1) von Oncor Inc. (Gaithersburg, MD, USA) verwendet. Vorgegossene GMA^{™} Wide Mini S-50 Gele und Spreadex EL 300 Wide Mini S-100 Gele wurden von Elchrom Scientific (Cham, Schweiz) gekauft. Vorgegossene Gele für die Polyacrylamidgelelektrophorese (Ready Gels 10%) waren von BioRad. [α-³²P] dCTP und Hybond-C Extra-Nitrozellulose-Membranen waren von Amersham International (Buckinghamshire, UK). DH5α Bakterien und 1kb-DNA-Leitern waren von GIBCO BRL, Life Technologies (Paisley, UK); QIAmp 96 DNA blood-Kits, Genomic tip-Kits, QIAquick Extraktions- und PCR Purifikations-Kits, QIAprep Spin Miniprep-Kits und QIAGEN Plasmid Midi-Kits waren von Qiagen.

### Methoden

Die in die Studie eingeschlossenen Individuen wurden für zwei bekannte DNA-Polymorphismen im Apolipoprotein E-Gen, die beide einen Aminosäurenaustausch bewirken (C112R, R158C), eine in der Schweizer Bevölkerung mit hoher Prävalenz vorkommende DNA-Mutation im Apolipoprotein B-100 Gen, die einen Aminosäurenaustausch (R3'500Q) verursacht, für einen neuen DNA-Polymorphismus im SREBP-1-Gen (G1028G), der zu keinem Aminosäureaustausch führt, und für einen neuen Polymorphismus im SREBP-2-Gen, der zu einem Aminosäureaustausch (A595G) führt, getestet. Eine Untergruppe dieser Personen, bei denen weitere Familienmitglieder mit Hypercholesterinämie untersucht wurden (SIPSHIP Studie), wurde auf das Vorhandensein von DNA-Restriktions-Fragmentlängen-Polymorphismen im LDL-Rezeptor-Gen untersucht, was die Durchführung von Kosegregationsstudien zur Bestätigung von LDL- Rezeptordefekten erlaubte. Individuen aus der SIPSHIP Studie wurden zusätzlich systematisch für Mutationen im LDLR-Gen getestet.

### 1. Lipoprotein Analysen

Nüchtern-Blutproben wurden von allen Personen, die in die Studie eingeschlossen wurden, abgenommen. Lipid- und Lipoproteinanalysen wurden am Zentrallaboratorium der Universitätskliniken Basel durchgeführt, mit Ausnahme einer Untergruppe aus der SIPSHIP Studie mit familiären Formen von Hyperlipoproteinämien, die bei Studieneintritt bereits behandelt waren und bei denen eine Auswaschperiode aufgrund ethischer Ueberlegungen nicht durchgeführt wurde. In dieser Untergruppe von Patienten wurden die Gesamtcholesterinkonzentrationen im Zentrallabor vor Beginn der medikamentösen Behandlung (pretreatment TC) bestimmt, oder die teilweise in anderen Laboratorien bestimmten Gesamtcholesterinkonzentrationen vor Behandlung wurden von ihren behandelnden Hausärzten erfragt und in die Analyse aufgenommen. LDL-Cholesterin (LDLC) wurde mittels Heparin präzipitiert (Merck, Darmstadt, Deutschland) und mit Hilfe der Friedewald-Formel errechnet. HDL-Cholesterin (HDLC) wurde präzipitiert mittels Phosphor-Wolfram-Säure und Magnesium-Ionen (Roche Diagnostics). Gesamtcholesterin (TC), LDLC und HDLC Plasmakonzentrationen wurden mittels der enzymatischen kolorimetrischen Cholesterin 4-Aminophenazon (PAP) - Methode von Roche Diagnostics auf einem Hitachi analyzer, Modell 737, gemessen.

### 2. DNA-Extraktionsmethode

Genomische Gesamt-DNA der in die Studie eingeschlossenen Personen wurde aus weissen Blutzellen mit Hilfe der Aussalzmethode(1), mit den früher beschriebenen Modifikationen(2), oder mittels der QIAmp^{™} 96 DNA blood-Kits von Qiagen extrahiert.

### 3. Einzel-Strang-Konformations-Polymorphismus (SSCP)

### a) Radioaktive Methode

Um LDL-Rezeptorgen-Mutationen nachzuweisen wurden alle 18 Exons des LDL-Rezeptor-Gens mittels der von Hobbs et al.(3)publizierten Oligonukleotide amplifiziert.

Zur Vervielfältigung von Exon 18c des SREBP-1-Gens inklusive der Exon-/Intron-Grenzen, um auch Spleiss-Mutationen zu entdecken, wurde das folgende Paar von Oligonukleotiden verwendet: S1.18cF (Seq. Id. Nr. 9): 5'-TTATTTATAATCTGGGTTTTGTGTC-3' und S1.18cR (Seq. Id. Nr. 10): 5'-GGGAAGAGCTAAGTTAAAAGTTGTG-3'. Um Exon 10 des SREBP-2-Gens inklusive dessen Exon-/Intron-Grenzen zu amplifizieren wurden die Oligonukleotide EcoRI S2.10F (Seq. Id. Nr. 15): 5'-CGGAATTCGCCAGTGACCATTAACACCTTTTGA-3' und EcoRI S2.10R (Seq. Id. Nr. 16): 5'-CGGAATTCTGCAGCAAGCCAGTCATCAGCAGCT-3' verwendet. Die PCR wurde in einem Endvolumen von 6µl in 1x PCR Puffer (Perkin Elmer) unter Verwendung von 1.0 U *Taq* Polymerase (Qiagen), 74 kBq [α-³²P] dCTP (Amersham) mit einer Endkonzentration von 1.5 mM MgCl₂, 420 µM jeder der vier dNTP (Qiagen) und 8.3 µM jeder der beiden Oligonukleotide durchgeführt.

Für SSCP des LDL-Rezeptor-Gens wurde genomische DNA (200ng) unter den folgenden PCR Bedingungen amplifiziert: 95°C, 180 Sek. (1 Zyklus); 95°C, 45 Sek.; 58°C, 30 Sek.; 72°C, 120 Sek. (29 Zyklen). Für SSCP der SREBP-1 und SREBP-2 Gene wurden 200ng genomischer DNA wie folgt PCR amplifiziert: 95°C, 180 Sek. (1 Zyklus); 95°C, 60 Sek.; 58°C, 30 Sek.; 72°C, 60 Sek. (30 Zyklen). Anschliessend an die PCR wurden 25 µl Denaturierungs-Puffer (95 % Formamid, 0.05 % Bromphenolblau, 0.05 % Xylencyanol, 20 mM EDTA) der PCR Mischung zugegeben. Nach 5 Minuten Denaturierung bei 95°C wurden 6 µl der Mischung auf ein 7 % Polyacrylamidgel (Acrylamid: Bisacrylamid Mischung 37.5:1), 2x TBE, 1.37 M Glyzerol, Geldicke 0.75 mm) geladen und das Gel wurde in 1x TBE Puffer bei 4°C in einem Kühlraum oder bei Raumtemperatur bei 15-20 V/cm für 12-16 Stunden laufen gelassen. Danach wurde das Gel mit Hilfe eines Vakuumtrockners bei 80°C eine Stunde lang getrocknet und Kodak X-OMAT AR Filme wurden bei Raumtemperatur 3-36 Stunden belichtet.

### b) Nicht-radioaktive Methode

Zur nicht-radioaktiven Identifizierung von Sequenzvariationen in Exon 10 des SREBP-2 Gens inklusive seiner Exon-/Intron-Grenzen wurden die Oligonukleotide *EcoR* I S2.10F und Eco R I S2.10R verwendet. Die PCR wurde in einem Endvolumen von 11 µl in 1x PCR Puffer (Qiagen) unter Verwendung von 1.0 U *Taq* Polymerase (Qiagen) und Endkonzentrationen von 1.5 mM MgCl₂, 909 µM jeder der vier dNTP (Qiagen), und 4.6 µM jeder der beiden Oligonukleotide durchgeführt. Genomische DNA (100ng) wurde unter den folgenden Bedingungen amplifiziert: 95°C, 180 Sek. (1 Zyklus); 95°C, 60 Sek.; 58°C, 30 Sek; 72°C, 60 Sek. (29 Zyklen). Anschliessend an die PCR wurden 25 µl Denaturierungs- bzw. Lade-Puffer (97 % Formamid, 0.05 % Bromphenolblau; 0.05 % Xylencyanol, 10mM NaOH) der PCR Mischung zugegeben. Nach 5 Minuten Denaturierung bei 92°C und sofortiger Abkühlung auf Eis für 10 Minuten wurden 6 µl der Mischung auf Elchrom GMA Wide Mini S-50 Gele geladen und mit 1x TAE Puffer (Puffer Temperatur 9°C) bei 6 V/cm in einer Elchrom Sea 2000 Elektrophorese Kammer für 14 Stunden laufen gelassen. Nach Entfernung des Stützplastiks wurde das Gel 40 Minuten lang in 50 ml SYBR^{®} Gold (Arbeitslösung nach Angaben des Herstellers Molecular Probes) in 0.75 x Standard TAE Puffer (4) auf einem Schüttler gefärbt. Nach 40-minütiger Entfärbung in 100 ml destilliertem Wasser auf einer Schüttelmaschine wurde das Gel analysiert mittels 302 nm UV Durchleuchtung und unter Verwendung eines Gel Doc 1000 Systems von BioRad digitalisiert.

### 4. Sequenzierung von LDL-Rezeptor-Gen-Mutationen und SREBP-1, Exon 18c, und SREBP-2, Exon 10,-Mutationen

Die entdeckten Sequenzvariationen wurden weiter analysiert mittels Subklonierung amplifizierter Exone und anschliessender Sequenzierung des Inserts. Beim LDL-Rezeptor-Gen wurde die PCR-Amplifizierung der entsprechenden Exone mittels der oben beschriebenen Oligonukleotide durchgeführt (3). Im SREBP-1-Gen wurde die PCR-Amplifikation des Exon 18c mittels Oligonukleotiden, die zusätzliche EcoR I-Schnittstellen besassen, durchgeführt; EcoR I.S1.18cF (Seq. Id. Nr. 11): 5'-CGGAATTCTGAAATTATTTATAATCTGGGTTTTGTGTC -3' und EcoR I.S1.18cR (Seq. Id. Nr. 12): 5'-CGGAATTCATCGGGGAAGAGCTAAG TTAAAAGTTGTG-3'. Im SREBP-2 Gen wurde die PCR Amplifikation des Exon 10 mittels EcoR I S2.10F: und EcoR I S2.10R: durchgeführt.

Die Amplifikationsreaktionen wurden in einem Endvolumen von 50 µl mit 1x PCR Puffer (Qiagen) unter Verwendung von 2.5 U *Taq* Polymerase (Qiagen) und Endkonzentrationen von 1.5 mM MgCl₂, 500 µM jeder der dNTP (Qiagen) und 2.0 µM jeder der beiden Oligonukleotide ausgeführt. Die folgenden Temperaturen wurden mittels eines Robocyclers erreicht: 95°C, 45 Sek.; 58°C, 30 Sek.; 72°C, 45 Sek. (30 Zyklen). Die amplifizierten Fragmente (50 µl) wurden auf ein 1% Agarosegel, das 0.6 µg/ml Ethidiumbromid enthielt, geladen, ausgeschnitten und mittels des QIAquick^{™} Extraktions-Kits (Qiagen) gereinigt. Das DNA-Fragment wurde mit 20 U EcoR I für mindestens 3 Stunden verdaut und mit Hilfe des QIAquick^{™} PCR Purifikations-Kits gereinigt. Der Vektor pcDNA 3.1 His A (3-5 µg) wurde mit 40 U EcoR I für drei Stunden verdaut. Im Anschluss daran wurden 20 U Kalb-Intestinal-Peptid (Roche Diagnostics) dazugegeben und für 1 Stunde bei 37°C inkubiert. Der Vektor wurde mit dem QIAquick^{™} PCR Purifikations-Kit gereinigt und mit 50 µl Wasser eluiert. Die Ligation wurde mittels des Ligations-Kits von Takara durchgeführt. Das gereinigte PCR Produkt (4 µl) und der gereinigte pcDNA3.1 His A Vektor (1 µl) wurden entsprechend den Empfehlungen des Herstellers ligiert und in *E.Coli* DH5α Bakterien (Life Technologies) mittels der Hitzeschockmethode (42°C für 45 Sek.) transformiert. Von Personen mit dem Wildtyp, gemäss den SSCP Resultaten, und von Personen mit der Sequenzvariation wurden 5-7 Kolonien ausgewählt und in 10 µl Wasser resuspendiert. Die Genotypisierung zur Festlegung des Vorhandenseins der Sequenzvariation wurde mit 2 µl der Bakteriensuspension und SSCP-Methoden, wie oben beschrieben, durchgeführt. Unabhängige Klone jeder der zwei Zustände (Wildtyp/Mutation) aus zwei unabhängigen PCR wurden sequenziert. Die DNA- Sequenzierung wurde durch die Microsynth AG durchgeführt mittels der Dideoxy-Ketten-Terminations-Methode. Um die Klone zu expandieren wurden 5 µl der übriggebliebenen Suspension zu 3 ml LB Medium, das 100 µg/ml Ampicillin enthielt, gegeben und über Nacht bei 37°C inkubiert. Von 1.5 ml der Bakteriensuspension wurde die Plasmid-DNA gereinigt unter Verwendung des QIAprep^{™} Spin Miniprep-Kits (Qiagen).

### 5. Test auf Apolipoprotein E-Mutationen, die die Plasmacholesterin-konzentration beeinflussen

Die beiden häufigen Apolipoprotein E Aminosäurepolymorphismen C112R und R158C wurden mittels PCR Amplifikation und anschliessender Verdauung mit *Hha* I bzw. des Isoschizomers *Cfo* I nach dem Protokoll von Hixson und Vernier (5) identifiziert.

### 6. Test auf Apolipoprotein B-Mutationen, die die Plasmacholesterin-konzentration beeinflussen

Drei unterschiedliche molekularbiologische Methoden wurden verwendet, um Mutationen zu suchen, die zu einem Aminosäureaustausch an Position 3'500 des Apolipoprotein B-Gens führen. Proben von Personen aus der SREAD Studie (2) mit Gesamtcholesterinkonzentrationen ≤ 4.5 mmol/L wurden gepoolt (25 Proben) und mittels der Methoden von Ruzicka et al., 1992(6)und von Schuster et al.(7) auf Mutationen getestet. Personen mit Gesamtcholesterinkonzentrationen > 4.5 mmol/L und positive Pools aus der SPREAD Studie, als auch alle anderen Proben, die bis 1996 untersucht wurden, wurden einzeln mittels allelspezifischer, asymmetrischer PCR, wie oben beschrieben (2), getestet. Von 1996 an wurde diese Methode durch eine site-directed Mutagenese-PCR-Technik, die eine *Msp* I Restriktionsschnittstelle bei Wildtypproben einführt, ersetzt. Die darauffolgende Verdauung mit *Msp* I(8) ermöglichte die Identifizierung von Patienten mit der R3'500Q Mutation.

### 7. Methoden zur Identifikation der SREBP-1, Exon 18c, und SREBP-2, Exon 10, -Polymorphismen mittels Restriktionsenzymverdauung

Im SREBP-1 Gen wurde das gesamte Exon 18c mittels des Primerpaars S1.18cF und S1.18cR amplifiziert; Exon 18c enthält den Polymorphismus, der eine variable *Xmn* I Schnittstelle generiert. Im SREBP-2 Gen wurde lediglich der 5' Teil des Exon 10 amplifiziert, der den Polymorphismus enthält, der eine variable *Msp* I Restriktions-schnittstelle generiert. Auf diese Weise wurde verhindert, dass weitere *Msp* I-Schnittstellen amplifiziert wurden, und ein komplexes Restriktionsmuster wurde vermieden. Beim SREBP-2 Gen wurden die folgenden Oligonukleotide verwendet: S2.10P.F (Seq. Id. Nr. 13):5'-GCCAGTGACCATTAACACCTTTTGA-3' und S2.10P.R (Seq. Id. Nr. 14):5'-TCGTCTTCAAAGCCTGCCTCAGTGGCTGGC-3'.

Zur Darstellung des SREBP-1-Polymorphismus wurden 80 ng genomischer DNA von den untersuchten Personen unter den nachfolgenden PCR Bedingungen amplifiziert: 95°C, 240 Sek. (1 Zyklus); 95°C, 60 Sek.; 55°C, 60 Sek.; 72°C, 90 Sek. (33 Zyklen). In einem Gesamtvolumen von 25 µl wurden 2.0 µM von jedem der beiden Oligonukleotide, 400 µM jeder der dNTP (Qiagen), 1x PCR Puffer (1.5 mM MgCl₂ Endkonzentration, Perkin Elmer) und 0.6 U *Tag* Polymerase (Qiagen) gemischt. Vom nicht-gereinigten Amplikon wurden 20 µl in 1x NE Puffer mittels 16-32 U *Xmn* I (New England Laboratories), 0.2 µl 10mg/ml BSA und einer Inkubationstemperatur von 37°C für 5 Stunden verdaut.

Zur Darstellung des SREBP-2 Polymorphismus wurden ca. 100 ng genomischer DNA mittels PCR unter den folgenden Bedingungen amplifiziert: 95°C, 30 Sek.; 58°C, 30 Sek; 72°C, 90 Sek. (30 Zyklen). In einem Gesamtvolumen von 25 µl wurden 1.37 µM von jedem der beiden Oligonukleotide, 390 µM jeder der dNTP (Qiagen), 1x PCR Puffer (1.5 mM MgCl₂ Endkonzentration, Perkin Elmer) und 0.75 U *Taq* Polymerase (Qiagen) gemischt. Vom resultierenden Amplikon wurden 20 µl in 1x NE Puffer mittels 16 U *Msp* I und einer Inkubationstemperatur von 37°C für 5 Stunden verdaut. Zur Identifikation der zwei Polymorphismen wurden 6-8 µl der verdauten Reaktionsgemische auf 10 % Polyacrylamid Ready Gele (BioRad) geladen und mit 1x TBE Puffer bei Raumtemperatur auf 18-22 V/cm für 25-35 Minuten laufen gelassen. Die Gele wurden daraufhin in einer 50 ml 0.5 µg/ml Ethidiumbromid-Lösung für 5 Minuten gefärbt und mittels eines Gel Doc 1000 Systems von BioRad bei einer Wellenlänge von 302 nm UV Licht digitalisiert.

### 8. Untersuchung auf LDL-Rezeptor-Mutationen als Ursache für erhöhte Plasmacholesterin-Konzentrationen

Bei einer Untergruppe von 48 Personen wurde die klinische Diagnose einer familiären Hypercholesterinämie aufgrund eines LDL-Rezeptor Defektes bestätigt mittels Kosegregationsstudien unter Verwendung von 10 verschiedenen RFLP im LDL-Rezeptorgen (9,10). Bei 110 von insgesamt 446 Familien wurden alle Exone des LDL-Rezeptorgens mittels SSCP (radioaktive Methode) und der publizierten Oligonukleotide (3) untersucht. Bei 22 Familien wurde das Vorhandensein von LDL-Rezeptor Mutationen durch Subklonieren und Sequenzieren von Exonen, die Sequenzvariationen aufwiesen, bestätigt.

### 9. Statistische Analyse: Populationsgenetik

Die Daten des Geneva Survey, einer Studie bei Schulkindern (13) und Daten aus der Swiss MONICA Studie (3,341 Individuen eingeschlossen (14)), wurden verwendet, um alters- und geschlechtsspezifische 90-er Perzentilen für Gesamtcholesterin und Triglyzeride in der Schweiz zu generieren. Alle Berechnungen wurden auf Mackintosh G3 Computern unter Verwendung der FileMaker^{®} Datenbank CARDIOFILE, der StatView^{®} und SuperANOVA^{®} Programme ausgeführt.

Individuen mit Plasmacholesterin-Konzentrationen unter der 90-er Perzentile wurden als normocholesterinämisch (NC) bezeichnet, Individuen mit Gesamtcholesterin-konzentrationen über der 90-er Perzentile als hypercholesterinämisch (HC). Bei beiden Gruppen wurde der Einfluss des Vorhandenseins der Apo E Mutationen C112R, R158C, und der neuen Aminosäurepolymorphismen im SREBP-2 Gen (A595G) und im SREBP-1 Gen (G1028G) mit Hilfe multivariater Testverfahren bestimmt.

### 10. Auswertung der nach obigen Angaben erhaltenen Resultate

### 10.1 Assoziation zwischen Polymorphismen in den SREBP-1 und -2 Genen mit Plasma- cholesterinkonzentrationen

### 10.1.1. Nachweis von Mutationen in den SREBP-1 and -2 Genen mit PIC-Werten über 0.25

Ein Personenkollektiv wurde auf das Vorhandensein von Sequenzvariationen mittels der Einzelstrang-Konformation-Polymorphismus-Methode (SSCP) untersucht. Ziel war der Nachweis von Polymorphismen, deren Vorkommen eine Häufigkeit erreicht, dass populationsgenetische Untersuchungen vorgenommen werden können. Dazu wurde ein Polymorphismusinformationsgehalt (PIC) - Wert von über 0.25 festgelegt. Die zwei durch die Einzelstrang-Konformation-Polymorphismus-Methode nachgewiesenen Sequenzvariationen, die eine im Exon 18c des SREBP-1 Gens und die andere im Exon 10 des SREBP-2 Gens, erfüllen diese Bedingung und wurden deshalb weitergehend charakterisiert. Exon 18c des SREBP-1 Gens und Exon 10 des SREBP-2 Gens von Personen, die die entsprechenden, vom Wildtyp abweichenden SSCP - Muster aufwiesen, wurden amplifiziert. Diese Exonsequenzen wurden subkloniert und sequenziert.

Figur 1A zeigt das Chromatogramm einer Person, die Träger eines DNA Polymorphismus an Aminosäureposition 1028 im Exon 18c des SREBP-1 Gens (G1028G) ist. Figur 1B zeigt das Chromatogramm einer Person, bei der ein DNA Polymorphismus, der zu einem Aminosäureaustausch an Position 595 im SREBP-2 Gen führt (A595G), nachgewiesen wurde.

Im SREBP-1 Gen fand sich eine Basensubstitution C → G im Exon 18c. Diese Basensubstitution führt nicht zu einem Aminosäureaustausch, generiert aber eine *Xmn* I Restriktionsschnittstelle (Figur 1A). Im SREBP-2 Gen wurde eine Basensubstitution C → G entdeckt. Diese Basensubstitution führt zu einem Austausch von Alanin zu Glycin in der Aminosäuresequenz und generiert eine zusätzliche Restriktionsschnittstelle *Msp* I (Figur 1).

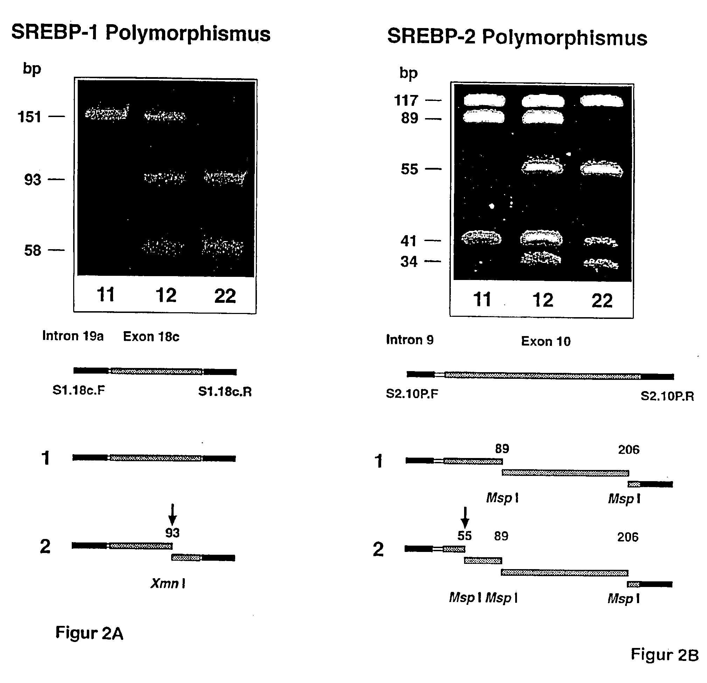

Die entsprechenden PIC - Werte, die aus allen eingeschlossenen Personen mit Ausnahme der verwandten Individuen aus der SIBSHIP Studie (N=2'446) errechnet wurden, betrugen 0.368 für den SREBP-1-Genpolymorphismus und 0.300 für den SREBP-2- Genpolymorphismus. Um grössere Kollektive hinsichtlich dieser Polymorphismen screenen zu können, wurde für jeden der beiden Polymorphismen eine Methode entwickelt, die auf einer PCR Amplifikation des entsprechenden DNA Abschnitts und darauffolgender Restriktionsenzymverdauung beruht (Figur 2). Weder der G1028G Polymorphismus noch der A595G Polymorphismus wichen signifikant vom Hardy - Weinberg Gleichgewicht ab (P>0.70 bzw. P> 0.10, wenn ein rezessiver Effekt zugrunde gelegt wurde). Bei HeLa Zellen wurde der G1028G Polymorphismus nur auf einem der beiden Allele entdeckt (heterozygot hinsichtlich des G1028G Polymorphismus (12)). Die A595G Mutation konnte bei HeLa Zellen nicht nachgewiesen werden (homozygot hinsichtlich des A595A Polymorphismus (11)).

### 10.1.2. Populationsgenetik

Insgesamt wurden 3'078 Individuen untersucht; 2600 Individuen, bei denen Gesamtcholesterinwerte ohne Behandlung gemessen wurden, wurden hinsichtlich der Mutationen und Polymorphismen in vier Genen getestet. Eine Untergruppe von 954 Personen setzte sich aus Zufallsstichproben von Querschnittsuntersuchungen zusammen (SPREAD, IDA), 318 waren nicht verwandte Individuen aus der SIBSHIP Studie (eine nicht betroffene Person pro Familie und alle Ehegatten, Schwager und Schwägerinnen, die genetisch unverwandt waren (REL)). Insgesamt 871 Personen wurden aus Patientenkollektiven mit primären und sekundären Hyperlipoproteinämien eingeschlossen. Alle 3'078 Individuen der Patienten- und Kontrollkollektive wurden auf das Vorhandensein der Mutation im Apo B-100 Gen, die zu einem Aminosäureaustausch bei Position 3'500 führt, getestet, um Patienten mit FDB in Kontrollkollektiven und den Patientenkollektiven mit Hyperlipoproteinämien zu identifizieren. Um Patienten mit familiärer Dysbetalipoproteinämie zu identifizieren, wurden alle 3'078 Individuen auf das Vorhandensein der Mutation im Apo E Gen bei Aminosäureposition 158 (E2 Allel) sowie auf das Vorhandensein der Mutation bei Position 112 (E4 Allel) untersucht. Das Vorhandensein von LDL-Rezeptor-Gendefekten führt generell zu einem signifikanten, d.h. zwei- oder dreifachen Anstieg der Gesamtcholesterinkonzentrationen, weshalb nur Patienten mit primären Formen von Hyperlipoproteinämien und deutlich erhöhten Gesamtcholesterinkonzentrationen auf das Vorhandensein dieser Mutationen hin untersucht wurden. Tabelle 1 zeigt eine Uebersicht über die verschiedenen Patienten- und Kontrollkollektive. Personen aus den Kontrollgruppen, die nachweislich unter bestimmten Störungen litten, die zu primären bzw. sekundären Formen von Hyperlipoproteinämie führen, wurden ebenfalls in die Patientenkollektive mit der entsprechenden Störung eingeschlossen. Somit ist die Summe der Personen aller Untergruppen grösser als die Anzahl der Individuen insgesamt (N=2'600). Tabelle 1 stratifiziert die Patienten- und Kontrollkollektive nach Individuen mit Gesamtcholesterinkonzentrationen unterhalb der 90er Perzentile (normocholesterinämisch, NC) und Individuen mit Gesamtcholesterinkonzentrationen oberhalb der 90er Perzentile (hypercholesterinämisch, HC).

Die in Tabelle 1 aufgeführten Patienten- und Kontrollkollektive wurden auf das Vorhandensein des beschriebenen Polymorphismus getestet mittels der oben erläuterten Methoden (Screenen von Personenkollektiven mit hohem Durchsatz).

Drei weitere Gene wurden untersucht: das Apo E Gen (Aminosäurepolymorphismen C112R und R158C), das Apo B-100 Gen (Mutation bei Aminosäureposition 3'500, R3'500Q). Im LDL-Rezeptor-Gen wurden Mutationen, die zu familiärer Hypercholesterinämie führen, mittels SSCP, darauffolgender Amplifikation der entsprechenden Exone, in denen Sequenzvariationen gefunden wurden, Subklonieren und Sequenzieren, identifiziert.

Insgesamt 3'078 Individuen wurden auf das Vorhandensein der Aminosäuresubstitution im Apo B Gen getestet (R3'500Q), sowie auf das Vorhandensein von zwei Aminosäurepolymorphismen im Apo E Gen (C112R oder E4 Allel, R158C oder E2 Allel), von denen bekannt ist, dass alle drei Mutationen die Plasmacholesterinkonzentration modifizieren.

Ausserdem sind 2'600 Personen hinsichtlich eines neuen DNA Polymorphismus im SREBP-1 Gen (G1028G) untersucht worden, der als Marker benutzt wurde. Alle 3'078 Personen wurden hinsichtlich eines neuen DNA Polymorphismus im SREBP-2 Gen (A595G) getestet, der zu einem Aminosäureaustausch führt.

Bei diesen Personen wurden die Plasmagesamtcholesterinkonzentrationen gemessen. Von den 3'078 eingeschlossenen Personen nahmen 478 lipidsenkende Medikamente bei Studieneintritt; Gesamtcholesterinkonzentrationen ohne Behandlung standen nicht zur Verfügung, weshalb diese Personen von weiteren Untersuchungen ausgeschlossen wurden. Von den übrigen 2'600 Personen wurden dann, wie beschrieben, Plasmagesamtcholesterinkonzentrationen ohne Behandlung, standardisiert für ein Alter von 50 Jahren, in die weitergehende Analyse aufgenommen. Tabelle 2 fasst die Resultate der Prävalenz der beiden Polymorphismen in den entsprechenden Untergruppen der Patienten- und Kontrollkollektive sowie die gemittelten Gesamtcholesterinkonzentrationen der verschiedenen Untergruppen im Verhältnis zum Vorhandensein des Polymorphismus zusammen.

Insgesamt zeigte sich ein hoch signifikanter, cholesterinsenkender Effekt der A595G Mutation im SREBP-2 Gen (Tabelle 2, N=2'600; P=0.0005). Dieser Effekt war noch ausgeprägter, falls die homozygoten Träger der C112R Mutation im Apo E Gen (E4/E4) (N=107) von der Untersuchung ausgeschlossen wurden (N=2'493; P<0.0001). Verwendete man nur genetisch unverwandte Personen für die Untersuchung, schloss also verwandte Personen aus der SIBSHIP Studie aus, sank die Wahrscheinlichkeit noch mehr, dass der Unterschied auf Zufall basierte (N=2'446, P=0.0003). Figur 3 zeigt die Analyse des Kollektivs unverwandter Personen (N=2'446), welches sich aus allen Individuen mit Ausnahme der genetisch verwandten aus der SIBSHIP Studie zusammensetzte, nach Stratifizierung hinsichtlich verschiedener Kriterien. Figur 3A and B veranschaulichen den Effekt der G1028G und A595G Polymorphismen bei zufällig ausgewählten Individuen. Zufallsstichproben (als solche festgelegt) hinsichtlich Hypercholesterinämie waren die SPREAD und IDA Studien sowie die Kollektive unverwandter, nicht betroffener Individuen (REL), und Personen, die aufgrund von möglichen Beeinträchtigungen der Gedächtnisfunktion (MCS) ausgewählt wurden. Aufgrund des Vorhandenseins einer Hypercholesterinämie wurden die restlichen Kollektive ausgewählt (nicht zufällig). Mittels Varianzanalysen (ANOVA, Scheffé Test) konnte ein signifikanter Effekt für den G1028G Polymorphismus nachgewiesen werden, wenn das Kollektiv hinsichtlich der Auswahlgruppe (zufällig/nicht zufällig) stratifiziert wurde (P=0.0164). Ebenso konnte der Effekt in beiden Gruppen bezüglich der A595G Mutation gezeigt werden; ANOVA ergab eine Wahrscheinlichkeit, dass der Unterschied auf Zufall basierte, von P<0.0001. Figuren 3C und D zeigen die Stratifizierung hinsichtlich der 90er Percentile (NC, HC). Beim G1028G Polymorphismus ergab sich ein signifikanter Effekt unter Anwendung von ANOVA (P=0.0088). Bei der A595G Mutation zeigte die Analyse nach Berücksichtigung des zusätzlichen Faktors ebenfalls eine Wahrscheinlichkkeit von P<0.0001.

Ausserdem konnte der bekannte, cholesterinmodifizierende Effekt der Apo E Gen Polymorphismen C112R (ε4) und R158C (ε2) im getesteten Untersuchungskollektiv gezeigt werden (N=2'600; P<0.0001).

Figuren 3, E und F, verdeutlichen die Gen - Gen - Interaktionen zwischen Apo E und den SREBP-1 und -2 Genen. Wurden alle 2'600 Individuen in die Untersuchung eingeschlossen, war kein signifikanter, cholesterinmodifizierender Effekt des G1028G Polymorphismus im SREBP-1 Gen nachweisbar. Nach Einbeziehen der Effekte der Apo E Gene in der Analyse, war für den G1028G Polymorphismus der Unterschied zwischen der homozygoten Form des Polymorphismus (22) und den anderen zwei Allelen (11/12) nicht signifikant (ANOVA, P=0.0722). Wurden jedoch homozygote oder heterozygote Träger der Apo E C112R (ε4) Mutation ausgeschlossen (N=761), war der Effekt des Fehlens des Wildtyp - Allels auf die Plasmagesamtcholesterinkonzentrationen hoch signifikant (N=1'839; P<0.0001). Beim A595G Polymorphismus war der Unterschied zwischen der homozygoten Form des Wildtyp (11) und der anderen beiden Allele (12/22) schon hoch signifikant (P=0.0002) nach Einbeziehen der Effekte der Apo E Gene in die Analyse. Wenn homozygote oder heterozygote Träger der Apo E C112R (ε4) Mutation ausgeschlossen wurden, sank die Wahrscheinlichkeit eines auf Zufall beruhenden Effekts weiter (P<0.0001).

Eine weitere Stratifizierung der Kollektive hinsichtlich der zugrunde liegenden Störungen, die entweder eine primäre oder sekundäre Hypercholesterinämie verursachen, ist in Tabelle 2 dargestellt. Die Resultate der Häufigkeitsberechnungen der beiden Polymorphismen (G1028G, A595G) entsprechend den verschiedenen Untergruppen finden sich in der ersten Zeile. Die Ergebnisse der gemittelten Gesamtcholesterinwerte ohne Behandlung in den verschiedenen Untergruppen, stratifiziert bezüglich des Vorhandenseins oder Fehlens der G1028G und A595G Polymorphismen, werden in der zweiten Zeile gezeigt (Tabelle 2). In den Untergruppen mit primären Hyperlipidämien hatte der Effekt der A595G Mutation statistische Relevanz in der Patientengruppe mit FDL (P=0.0020) und in der Patientengruppe mit primärer Hypercholesterinämie (PHC). Bei diesen Patienten waren Mutationen in den Apo E, Apo B und LDL Rezeptor Genen ausgeschlossen worden, obgleich ein autosomal-dominant oder -rezessiv vererbter Gendefekt als Ursache für Hypercholesterinämie vermutet wurde. In dem letztgenannten Kollektiv war die Prävalenz des Wildtyp (11) Allels signifikant höher (9.38%) als in der Summe der anderen Kollektive (6.69%) (P=0.0328).

Hinsichtlich der Kollektive mit sekundären Hyperlipoproteinämien (hierzu gehören sowohl als NC als auch als HC klassifizierte Personen), konnte bei Männern mit Diabetes mellitus und normalen Plasmatriglyceridkonzentrationen (TG<2.3mmol/L) ein signifikanter Unterschied zwischen A595A (11) und A595G (12/22) positiven Individuen festgestellt werden (P=0.0018). Bei 11.6% der Individuen mit sekundären Hyperlipoproteinämien waren die Plasmatriglyceridkonzentrationen erhöht.

Im SREBP-1 Gen betrug die Prävalenz des Wildtyp Allels im homozygoten Zustand (11) 40.35%, die Prävalenz des G1028G Polymorphismus im heterozygoten Zustand (12) 45.62% und im homozygoten Zustand (22) 14.04% (N=2'600). Im SREBP-2 Gen war die Prävalenz des Wildtyps im homozygoten Zustand (11) 6.69%, die Prävalenz der A595G Mutation im heterozygoten Zustand (12) 35.15% und im homozygoten Zustand (22) 58.15% (N=2'600).

Um den Effekt der entdeckten DNA- und Aminosäurepolymorphismen auf die Plasmagesamtcholesterinkonzentrationen zu erklären, wurden 3'078 Individuen molekulargenetisch untersucht. Bei 2'600 Personen konnten sowohl demographische als auch klinische Daten ergänzt werden mit Angaben zu Alter, Geschlecht, Gesamtcholesterinkonzentrationen ohne Behandlung mit lipidsenkenden Medikamenten zum Zeitpunkt der Cholesterinbestimmung, Genotyp hinsichtlich des Apo E Aminosäurepolymorphisms (C112R oder ε4 Allel, R158C oder ε2 Allel), und zur Apo B100 Mutation, die zuerst als ursächlich für FDB (R3'500Q) entdeckt wurde. Der R158C Aminosäurepolymorphismus hat im homozygoten Zustand einen cholesterinmodifizierenden Effekt in der untersuchten Population (P<0.0001). Bei Personen, die Träger der R3'500Q Mutation (FDB) sind, waren die Gesamtcholesterinkonzentrationen erhöht im Vergleich zu Personen ohne Apo B Defekt (P<0.0001). Bei Personen mit nachgewiesenen LDL Rezeptor Mutationen (FHM) waren die gemittelten Gesamtcholesterinkonzentrationen im Vergleich zu den Kontrollen erhöht (P<0.0001). Insgesamt beeinflusst der im SREBP-1 Gen entdeckte DNA Polymorphismus (G1028G) die Plasmagesamtcholesterinkonzentrationen bei dieser Personengruppe nicht signifikant. In Kombination mit dem Vorhandensein der Apo B R3'500Q Mutation war der G1028G (22) Polymorphismus jedoch signifikant mit einem Anstieg der Plasmacholesterin-Konzentrationen assoziiert (P=0.0097). Eine weitergehende Stratifizierung der an der Studie beteiligten Personen entsprechend der zugrunde liegenden genetischen Störungen oder der klinischen Diagnose bestätigte den Effekt des neuen SREBP-2 Aminosäurepolymorphismus auf Plasmagesamtcholesterinkonzentrationen bei fast allen Gruppen, aber keiner der Unterschiede erreichte statistische Signifikanz ausser bei der Patientengruppe mit familiärer Dysbetalipoproteinämie, FDL, und bei Personen, die an Hypercholesterinämie aufgrund unbekannter Gendefekte leiden (PHC).

### 10.2. Assoziation der A595G Mutation mit seniler Demenz vom Alzheimer Typ

Ein anderes bemerkenswertes Resultat der vorliegenden Studie ist die signifikante Differenz der Prävalenz des Wild-Typ Allels (A595A) verglichen mit der Aminosäuresubstitution (A595G), beim Vergleich eines klinisch diagnostizierten Alzheimerpatientenkollektivs mit der Prävalenz in der Allgemeinbevölkerung (Tabelle 2, 2.4% versus 7.0%; P= 0.0234).

### 10.3. Assoziation des G1028G Polymorphismus mit einem fehlenden Anstieg der Plasmalipid-Konzentration nach Gabe von Proteasehemmer bei HIV-Patienten

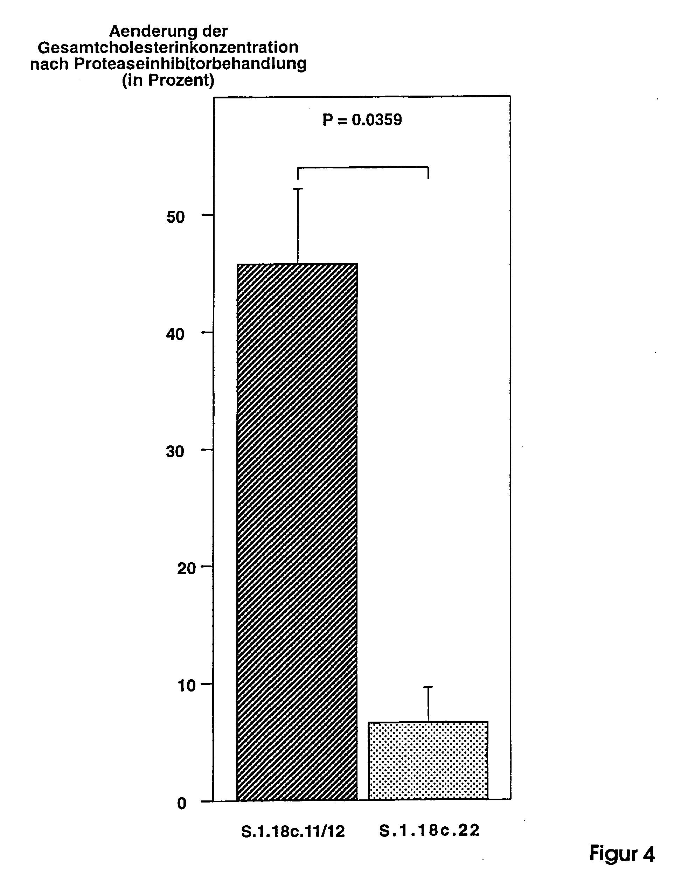

Die Resultate betreffend Prävalenzunterschiede sind ebenfalls in Tabelle 2 dargestellt (P=0.0339). Figur 4 zeigt die prozentuale Aenderung der Plasmacholesterinspiegel vor und nach Gabe von Proteasehemmern in Abhängigkeit vom G1028G Polymorphismus.

### 11. Studie umfassend den Polymorphismus in Exon 6 von SREBP-2

### 11.1. Grundlagen

### Probanden

Es wurden insgesamt 1'081 Probanden aus denselben Gruppen wie bereits oben ausgeführt (711 aus der SPREAD Studie, 346 aus der IDA-Studie sowie 24 aus einem prospektiv untersuchten Kollektiv von in Basel Verstorbenen (PATH-Studie)) in diese Untersuchung aufgenommen.

### Material

Neben den bereits beschriebenen Materialien wurde zusätzlich das Restriktionsenzym Dde I (New England Biolabs) verwendet.

### Methoden

Die in die Studie eingeschlossenen Probanden wurden zusätzlich für eine weitere Mutation im SREBP-2-Gen (Exon 6), die zu einem Aminosäureaustausch (R371K) führt, getestet.

Die SREBP-2 R371K Mutation wurde im wesentlichen wie bereits beschrieben untersucht. Insbesondere wurde für die Lipoprotein Analysen, die DNS-Extraktionsmethode sowie für die Bestimmung des Einzel-Strang-Konformations-Polymorphismus (SSCP) nach der nicht-radioaktive Methode wie oben beschrieben vorgegangen.

### 11.2. Sequenzierung der SREBP-2, Exon 6-Mutation

Diese wurde wie bereits beschrieben durchgeführt, mit den folgenden Modifikationen: Im SREBP-2 Gen wurde die PCR-Amplifikation des Exons 6 mittels der Oligonukleotide EcoR I.S2.6F (Seq. Id. Nr. 17): 5'-CGGAATTCTGGTCTCACTGTGTTTTCACTCATC-3'und EcoR I.S2.6R (Seq. Id. Nr. 18): 5'-CGGAATTCGCCAGGGCTGACAAGCCTTTTCTCA-3' durchgeführt. Die Amplifikationsreaktion wurde in einem Endvolumen von 50µl mit 1x Puffer (Qiagen) unter Verwendung von 0.4 U *Taq* Polymerase (Qiagen) und Endkonzentrationen von 3.5 mM MgCl₂, 455 µM jeder der dNTP (Qiagen) und 2.0 µM jeder der beiden Oligonukleotide bei den folgenden Temperaturen ausgeführt: 94°C (45"); 56°C (30"); 72°C (1'); 32 Zyklen. Die amplifizierten Fragmente wurden mittels Subklonierung und anschliessender Sequenzierung des Inserts (wie bereits beschrieben) analysiert.

### 11.3. Methoden zur Identifikation der SREBP-2, Exon 6-Mutation mittels Restriktionsenzymverdauung

Zur Darstellung der SREBP-2-Mutation (Exon 6, R371K) wurden ca. 100 ng genomischer DNS mittels der bereits oben beschriebenen Methoden unter Verwendung der Oligonukleotide *EcoR* I.S2.6F und EcoR I.S2.6R amplifiziert. 20µl wurden in 1x NE Puffer mittels 7 U *Dde* I und einer Inkubationstemperatur von 37°C für 5 Stunden verdaut. Zu dem verdauten Reaktionsgemisch wurden 4µl 5x nicht-denaturierender Ladepuffer (Elchrom) dazugegeben. 7µl dieser Mischung wurden auf Spreadex Wide-Mini S-100 Gele (Elchrom) geladen, bei 55°C auf 10V/cm für 25-45 Minuten laufen gelassen, mit Ethidiumbromid gefärbt (40 Minuten), entfärbt mit destilliertem Wasser (40 Minuten), und mittels eines Gel Doc 1000 Systems digitalisiert.

### 11.4. Statistische Methoden

Zum Vergleich der Prävalenzen von Sequenzvariationen in den SREBP-1 und -2 Genen wurde der Chi-Quadrat-Test angewandt.

### 11.5. Auswertung der nach obigen Angaben erhaltenen Resultate

Der Altersmedian bei den 698 Probanden der SPREAD-Studie betrug 20.5 Jahre (Altersbereich 18.8 - 43.7 Jahre), der Altersmedian bei den 370 Probanden der IDA-Studie 74.5 Jahre (Altersbereich 47.0 - 95.4 Jahre). Der Vergleich der zwei Gruppen von Probanden, die nicht selektioniert, aber unterschiedlichen Alters waren, zeigte statistisch signifikante Unterschiede im Vorhandensein von SREBP-1 und SREBP-2 Mutationen.

Die Prävalenz des Fehlens des SREBP-1c-G1028G Polymorphismus in homozygoter Form (d.h. Genotyp 11/12) bei den Probanden der SPREAD Studie betrug 622/711 (87.5 %) gegenüber nurmehr 304/367 (82.8%) bei den Probanden der IDA/PATH Studie. Dies ergibt einen Unterschied von absolut -4.7% (bzw. relativ -5.4%) mit einem P-Wert von 0.038 (Chi-Quadrat Test).

Die Prävalenz des Fehlens des SREBP-2 A595G Polymorphismus in homozygoter Form (d.h. Genotyp 11/12) bei den Probanden der SPREAD Studie betrug 305/711 (43.0%) gegenüber nurmehr 135/370 (36.5%) bei den Probanden der IDA/PATH Studie. Dies ergibt einen Unterschied von absolut -6.8% (bzw. relativ -15.1%) mit einem P-Wert von 0.041.

Die Prävalenz der SREBP-2 R371K Mutation bei den Probanden der SPREAD Studie betrug 19/698 (2.7%) gegenüber nurmehr 3/370 (0.8%) bei den Probanden der IDA/PATH Studie. Dies ergibt einen Unterschied von absolut -1.9 (bzw. relativ -70.4%) mit einem P-Wert von 0.036.

### 11.6. Diskussion

Die Unterschiede in den Prävalenzen in den Gruppen von jüngeren bzw. älteren Probanden können nur durch Unterschiede in der Mortalität erklärt werden, da beide Stichproben aus derselben Bevölkerung gezogen wurden.

Bei der IDA/PATH-Studienpopulation mit einem Altersmedian von 74.5 Jahren sind demnach bereits zahlreiche Probanden verstorben, die Träger des SREBP-2 G1'028G Genotyps 11/12 sind: 316 Träger wurden in dieser Gruppe aufgrund der Daten der SPREAD-Studie erwartet, aber nur 304 Probanden wurden mit diesem Genotyp (11/12) beobachtet, 12 Probanden fehlen demnach in dieser Gruppe.

Dasselbe gilt für die Träger des SREBP-2 A595G Genotyps 11/12: 159 Träger wurden in dieser Gruppe aufgrund der Daten der SPREAD-Studie erwartet, aber nur 135 Probanden wurden mit diesem Genotyp (11/12) beobachtet, 24 Probanden fehlen demnach in dieser Gruppe.

Ebenfalls gilt dies für die selteneren Träger der SREBP-2 R371K-Mutation: 10 Träger wurden in dieser Gruppe aufgrund der Daten der SPREAD-Studie erwartet, aber nur 3 Probanden wurden mit dieser Mutation beobachtet, 7 Probanden fehlen demnach in dieser Gruppe.

Eine Erklärung für die signifikant niedrigere Prävalenz bestimmter Sequenzvariationen in SREBP-1 und SREBP-2 ist die erhöhte Mortalität bei Trägern der Genotypen SREBP-1.18c (11/12), SREBP-2.10 (11/12) und den Trägern der SREBP-2 R371K Mutation. Dies kann beispielsweise durch die bereits beschriebene Assoziation zur Erhöhung des Plasmacholesterinspiegels mit der Folge einer koronaren Herzkrankheit bedingt sein, aber auch durch das überproportionale Auftreten von Krankheiten, wie beispielsweise der senilen Demenz vom Alzheimer Typ, wie ebenfalls bereits beschrieben, durch die beiden genannten oder durch Kombinationen mit weiteren Risikofaktoren.

**Tabelle 1**

| Kollektiv | Studien | Beschreibung des Kollektivs | Alter | Geschlecht | Alle Individuen | | | Normocholesterinämische Individuen¹⁾ | | | Hypercholesterinämische Individuen¹⁾ | | |
|---|---|---|---|---|---|---|---|---|---|---|---|---|---|
| | | | Mittelwert | m / (m+w) | A | TC | | A | TC | | A | TC | |
| | | | | | Mittelwert (±1SD) | | | Mittelwert (±1SD) | | | Mittelwert (±1SD) | | |
| **Alle Individuen** | | | | | | | | | | | | | |
| TTL | SPREAD,SIBSHIP IDA,MCS,STARTER | Alle Altersgruppen | 45.55 | 0.66 | 2'600 | 6.84 | ±2.52 | 1'980 | 5.80 | ±1.32 | 620 | 10.14 | ±2.58 |
| | | | | | | | | | | | | | |

| **Kollektive zufällig ausgewählter Individuen** | | | | | | | | | | | | | |
|---|---|---|---|---|---|---|---|---|---|---|---|---|---|
| RDM.YNG | SPREAD | Zufallsstichprobe (jung) | 20.77 | 1.00 | 630 | 5.12 | ±0.88 | 612 | 5.05 | ±0.78 | 18 | 7.40 | ±1.17 |
| RDM.ELD | IDA | Zufallsstichprobe (älter) | 75.98 | 0.67 | 324 | 6.46 | ±1.49 | 316 | 6.39 | ±1.45 | 8 | 8.93 | ±1.01 |
| | | | | | | | | | | | | | |

| **Kollektive von Individuen mit Störungen, die primäre Hyperlipoproteinämien verursachen** | | | | | | | | | | | | | |
|---|---|---|---|---|---|---|---|---|---|---|---|---|---|
| FDL | SPREAD,SIBSHIP IDA,MCS,STARTER | Apolipoprotein E Defekt (R158C, homozygot) | 46.17 | 0.76 | 46 | 9.90 | ±4.10 | 15 | 5.27 | ±1.43 | 31 | 12.15 →FDL | ±2.89 |
| FDB | SPREAD,SIBSHIP IDA,MCS,STARTER | Apolipoprotein B Defekt (R3'500Q, heterozygot) | 41.49 | 0.54 | 37 | 9.046 | ±1.341 | 5 | 6.93 | ±0.86 | 32 | 9.38 →FDB | ±1.08 |
| FHM | SIBSHIP | LDLR Defekt (molekular- | 34.87 | 0.51 | 74 | 10.93 | ±2.43 | | | | 74 | 10.93 | ±2.43 |
| | | genet. nachgewiesen) | | | | | | | | | | →FH | |
| PHC | SIBSHIP | Primäre, isolierte | 40.58 | 0.55 | 341 | 9.90 | ±2.62 | - | - | | 341 | 9.90 | ±2.62 |
| | | Hypercholesteriämie²⁾ | | | | | | | | | | | |
| PCH | SIBSHIP | Primäre, kombinierte Hyperlipoproteinämie²⁾ | 47.48 | 0.82 | 85 | 9.98 | ±2.22 | - | - | - | 85 | 9.98 →FCH | ±2.22 |
| REL | SIBSHIP | Verwandte, nicht betroffen | 40.19 | 0.54 | 318 | 6.39 | ±1.19 | 310 | 6.35 | ±1.17 | 8 | 8.11 | ±0.34 |

| **Kollektive von Individuen mit Störungen, die sekundäre Hyperlipoproteinämien verursachen⁴** | | | | | | | | | | | | | |
|---|---|---|---|---|---|---|---|---|---|---|---|---|---|
| DIA | STARTER,SIBSHIP IDA,MCS | Diabetes mellitus (GlucosePlasma >7.8mmol/L) | 50.96 | 0.59 | 229 | 6.48 | ±1.97 | 191 | 5.87 | ±1.21 | 38 | 9.55 → SHL | ±2.20 |
| RIN | STARTER,SIBSHIP IDA,MCS | Niereninsuffizienz (Klärunger < 50ml/min.)) | 76.63 | 0.44 | 131 | 7.17 | ±2.68 | 115 | 6.83 | ±2.58 | 16 | 9.65 → SHL | ±2.07 |
| LIV | STARTER,SIBSHIP IDA,MCS | Alkoholkonsum >60g/T und/oder γ-GT >664/l | 53.32 | 0.85 | 151 | 8.22 | ±2.89 | 86 | 6.49 | ±1.25 | 65 | 10.52 → SHL | ±2.84 |
| HTH | STARTER,SIBSHIP IDA,MCS | Schilddrüsenunterfunktion (TSH > 4.0mIU/L) | 59.53 | 0.15 | 102 | 6.54 | ±1.97 | 86 | 5.92 | ±1.02 | 16 | 9.86 → SHL | ±2.49 |

| **Kollektive von Individuen mit Störungen, die möglicherweise mit SREBP-1 und/oder -2- in Zusammenhang stehen** | | | | | | | | | | | | | |
|---|---|---|---|---|---|---|---|---|---|---|---|---|---|
| MEM.TTL | MCS | Erwachsene (alle Altersgruppen) | 70.63 | 0.46 | 413 | 6.24 | ±1.67 | 387 | 6.09 | ±1.61 | 26 | 8.39 | ±0.93 |
| MEM.DAT | | Demenz vom Alzheimer Typ (MMS < 26) | 73.50 | 41.2 | 165 | 6.16 | ±1.31 | 157 | 6.06 | ±1.25 | 8 | 8.17 | ±0.61 |
| HIV.STB | STARTER | TC, nicht ansteigend unter Proteaseinhibitoren⁵⁾ | 34.24 | 80.0 | 25 | 4.79 | ±1.31 | 24 | 4.70 | ±1.25 | 1 | 7.05 | |
| HIV.INC | | TC, ansteigend unter Protease Inhibitoren⁵⁾ | 38.06 | 85.0 | 20 | 4.52 | ±0.91 | 20 | 4.52 | ±0.91 | | | |

| | | | | | | | | | | | | | |
|---|---|---|---|---|---|---|---|---|---|---|---|---|---|
| ¹⁾Normocholesterinämisch: Plasmacholesterin < 90er Percentile, entsprechend Alter und Geschlecht; hypercholesterinämisch: Plasmacholesterin > 90er Percentile, entsprechend Alter und Geschlecht ²⁾Zugrunde liegende molekulare Defekte unbekannt ³⁾Nur unverwandte Individuen (eine nicht betroffene Person pro Familie und alle Ehegatten, Schwager und Schwägerinnen genetisch unverwandt) ⁴⁾Kombinierte Kollektive aus Individuen, die aufgrund von sekundären Hyperlipoproteinämien ausgewählt wurden, und Individuen aus anderen Kollektiven ⁵⁾HIV positive Individuen, bei denen die Plasmagesamtcholesterinkonzentrationen nicht (STB) oder nach Verabreichung von Proteaseinhibitoren ansteigen (INC) | | | | | | | | | | | | | |

**Tabelle 2**

| Kollektiv | | | **SREBP-1 Polymorphismus (G1028G)** | | | | | | | **SREBP-2 Polymorphismus (A595G)** | | | | | | | |
|---|---|---|---|---|---|---|---|---|---|---|---|---|---|---|---|---|---|
| | | **11** | | **12** | | **22** | | **P** | **P** | **11** | | **12** | | **22** | | **P** | **P** |
| | Prävalenz¹⁾ | | | | | | | | ΔPR³⁾ | | | | | | | | ΔPR⁴⁾ |
| TC Mittelwert²⁾ | | | | | | | | ΔTC⁵⁾ | | | | | | | | ΔTC⁶⁾ | |
| **Alle Individuen** | | | | | | | | | | | | | | | | | |
| TTL | PR | 40.35 | (1'049) | 45.62 | (1'186) | 14.04 | (365) | | | 6.69 | (174) | 35.15 | (914) | 58.15 | (1'512) | | |
| | TC | 6.74 | ±2.47 | 6.88 | ±2.53 | 6.99 | ±2.61 | 0.2309 | | 7.47 | ±3.48 | 6.83 | ±2.57 | 6.77 | ±2.34 | 0.0005 | |

| **Kollektive zufällig ausgewählter Individuen** | | | | | | | | | | | | | | | | | |
|---|---|---|---|---|---|---|---|---|---|---|---|---|---|---|---|---|---|
| RDM.YNG | PR | 41.75 | (263) | 45.71 | (288) | 12.54 | (79) | | 0.3270 | 5.87 | (37) | 36.51 | (230) | 57.62 | (363) | | 0.3445 |
| | TC | 5.04 | ±0.87 | 5.15 | ±0.90 | 5.24 | ±0.87 | 0.1735 | | 5.15 | ±0.85 | 5.06 | ±0.91 | 5.15 | ±0.87 | 0.7943 | |
| RDM.ELD | PR | 41.36 | (134) | 40.74 | (132) | 17.90 | (58) | | 0.0324 | 5.56 | (18) | 30.25 | (98) | 64.20 | (208) | | 0.3815 |
| | TC | 6.50 | ±1.55 | 6.41 | ±1.41 | 6.36 | ±1.53 | 0.5796 | | 6.77 | ±1.51 | 6.55 | ±1.66 | 6.39 | ±1.40 | 0.3525 | |

| **Kollektive von Individuen mit Störungen, die primäre Hyperlipoproteinämien verursachen** | | | | | | | | | | | | | | | | | |
|---|---|---|---|---|---|---|---|---|---|---|---|---|---|---|---|---|---|
| FDL | PR | 32.26 | (10) | 41.94 | (13) | 25.81 | (8) | | 0.0578 | 9.68 | (3) | 16.13 | (5) | 74.19 | (23) | | 0.5034 |
| | TC | 12.63 | ±2.78 | 11.54 | ±3.37 | 12.96 | ±2.15 | 0.4351 | | 16.89 | ±3.45 | 10.62 | ±1.76 | 12.01 | ±2.47 | 0.0020 | |
| FDB | PR | 33.33 | (12) | 38.89 | (14) | 16.67 | (6) | | 0.4401 | 8.33 | (3) | 44.44 | (16) | 36.11 | (13) | | 0.5412 |
| | TC | 9.99 | ±0.98 | 9.06 | ±1.12 | 8.92 | ±0.68 | 0.2393 | | 8.63 | ±0.0.22 | 9.21 | ±1.09 | 9.77 | ±1.01 | 0.1989 | |
| FHM | PR | 33.78 | (25) | 48.65 | (36) | 17.57 | (13) | | 0.3753 | 8.11 | (6) | 35.14 | (26) | 56.76 | (42) | | 0.6210 |
| | TC | 11.55 | ±1.98 | 10.56 | ±2.09 | 10.74 | ±3.58 | 0.7579 | | 11.15 | ±2.34 | 11.06 | ±2.13 | 10.81 | ±2.59 | 0.8118 | |
| PHC | PR | 38.12 | (130) | 48.09 | (164) | 13.78 | (47) | | 0.8842 | 9.38 | (32) | 37.24 | (127) | 53.37 | (182) | | 0.0328 |
| | TC | 9.74 | ±2.31 | 10.04 | ±2.97 | 9.85 | ±2.06 | 0.8737 | | 10.89 | ±4.82 | 9.90 | ±2.20 | 9.72 | ±2.31 | 0.0240 | |
| PCH | PR | 41.18 | (35) | 43.53 | (37) | 15.29 | (13) | | 0.7347 | 10.59 | (9) | 36.47 | (31) | 52.94 | (45) | | 0.1439 |
| | TC | 9.97 | ±2.46 | 9.81 | ±1.82 | 10.50 | ±2.69 | 0.3650 | | 9.71 | ±2.23 | 10.35 | ±2.98 | 9.78 | ±1.53 | 0.7019 | |
| REL | PR | 39.62 | (126) | 46.86 | (149) | 13.52 | (43) | | 0.7772 | 6.60 | (21) | 33.33 | (106) | 60.06 | (191) | | 0.9462 |
| | TC | 6.38 | ±1.16 | 6.44 | ±1.09 | 6.27 | ±1.55 | 0.4679 | | 6.43 | ±1.10 | 6.33 | ±1.26 | 6.42 | ±1.16 | 0.8984 | |

| **Kollektive von Individuen mit Störungen, die sekundäre Hyperlipoproteinänden verursachen** | | | | | | | | | | | | | | | | | |
|---|---|---|---|---|---|---|---|---|---|---|---|---|---|---|---|---|---|
| DIA | PR | 39.30 | (90) | 43.67 | (100) | 17.03 | (39) | | 0.1723 | 6.55 | (15) | 38.86 | (89) | 54.59 | (125) | | 0.9282 |
| | TC | 6.47 | ±1.78 | 6.44 | ±1.88 | 6.62 | ±2.57 | 0.6233 | | 6.48 | ±1.69 | 6.47 | ±1.92 | 6.49 | ±2.05 | 0.9985 | |
| RIN | PR | 40.46 | (53) | 45.80 | (60) | 13.74 | (18) | | 0.9197 | 6.11 | (8) ±2.04 | 40.46 | (53) ±3.42 | 53.44 | (70) ±2.07 | | 0.7832 |
| | TC | 7.27 | ±3.37 | 7.15 | ±2.01 | 6.96 | ±2.49 | 0.7193 | | 7.44 | | 7.14 | | 7.16 | | 0.7692 | |
| LIV | PR | 38.51 | (57) | 47.30 | (70) | 14.19 | (21) | | 0.9567 | 8.11 | (12) | 32.43 | (48) | 61.49 | (91) | | 0.5249 |
| | TC | 8.14 | ±2.55 | 8.53 | ±3.31 | 7.92 | ±2.11 | 0.6011 | | 9.81 | ±4.78 | 8.02 | ±3.06 | 8.25 | ±2.43 | 0.0696 | |
| HTH | PR | 37.63 | (35) | 46.24 | (43) | 16.13 | (15) | | 0.5545 | 6.45 | (6) | 24.73 | (23) | 68.82 | (64) | | 0.9246 |
| | TC | 6.17 | ±1.80 | 6.55 | ±1.87 | 6.00 | ±1.29 | 0.2678 | | 7.46 | ±1.56 | 7.09 | ±2.78 | 6.00 | ±1.11 | 0.1174 | |

| **Kollektive von Individuen mit Störungen, die möglicherweise mit SREBP-1 und/oder -2-** In **Zusammenhang stehen** | | | | | | | | | | | | | | | | | |
|---|---|---|---|---|---|---|---|---|---|---|---|---|---|---|---|---|---|
| TTL - DAT | PR | 40.16 | (978) | 45.59 | (1'110) | 14.25 | (347) | | | 6.98 | (170) ±3.52 | 34.99 | (852) ±2.63 | 58.03 | (1'413) ±2.39 | | |
| | TC | 6.78 | ±2.53 | 6.82 | ±2.58 | 7.05 | ±2.63 | 0.2225 | | 7.48 | | 6.88 | | 6.81 | | 0.0018 | |
| DAT | PR | 43.03 | (71) | 46.06 | (76) | 10.91 | (18) | | 0.2318 | 2.42 | (4) | 37.58 | (62) | 60.00 | (99) | | 0.0234 |
| | TC | 6.14 | ±1.22 | 6.24 | ±1.28 | 5.92 | ±1.78 | 0.4050 | | 7.44 | ±1.19 | 6.14 | ±1.34 | 6.12 | ±1.28 | 0.0487 | |
| HIV.STB | PR | 52.00 | (13) | 28.00 | (7) | 20.00 | (5) | | | 16.00 | (4) | 36.00 | (9) | 48.00 | (12) | | |
| | TC | 4.85 | ±1.53 | 4.27 | ±0.59 | 5.37 | ±1.37 | 0.2772 | | 3.99 | ±0.62 | 4.77 | ±1.47 | 5.08 | ±1.32 | 0.1855 | |
| HIV.INC | PR | 40.00 | (8) | 60.00 | (12) | 0.00 | (0) | | 0.0339 | 5.00 | (1) | 45.00 | (9) ±0.26 | 50.00 | (10) ±0.98 | | 0.2433 |
| | TC | 4.26 | ±1.03 | 4.70 | ±0.82 | - | - | - | | 2.13 | | 4.80 | | 4.51 | | 0.0036 | |

| | | | | | | | | | | | | | | | | | |
|---|---|---|---|---|---|---|---|---|---|---|---|---|---|---|---|---|---|
| ¹⁾ Prävalenz (=PR) in Prozent, (Anzahl der Individuen) ²⁾ Mittelwert der Plasmagesamtcholesterinkonzentrationen (=TC) in mmol/L, (±SD) ³⁾ ΔPR = PR11/12 vs. PR22; Signifikanzhöhe (P) des Unterschieds zwischen der Prävalenz des Kollektivs (PR.Kollektiv) vs. Prävalenz aller Individuen (PR.TTL) minus Prävalenz des entsprechenden Kollektivs (PR.Kollektiv): (P von PR.Kollektiv vs. (PR.TTL- PR.Kollektiv) ⁴⁾ ΔPR = PR11 vs. PR12/22; Signifikanzhöhe (P) des Unterschieds zwischen der Prävalenz des Kollektivs (PR.Kollektiv) vs. Prävalenz aller Individuen (PR.TTL) minus Prävalenz des entsprechenden Kollektivs (PR.Kollektiv): (P von PR.Kollektiv vs. (PR.TTL - PR.Kollektiv) ⁵⁾ ΔTC = TC11/12 vs. TC22 / ⁶⁾ ΔTC = TC11 vs. TC12/22 | | | | | | | | | | | | | | | | | |

### Literaturangaben:

(Literatur wird im Text direkt zitiert oder aber es wird auf die unten angeführten Dokumente durch Nennung der entsprechenden Zitatnummer (in Klammer) verwiesen)
1. Miller, S. A. 1988. A simple salting out procedure for extracting DNA from human nucleated cells. Nucleic Acids Res. 16:1215.
2. Miserez, A. R., R. Laager, N. Chiodetti, und U. Keller. 1994. High prevalence of familial defective apolipoprotein B-100 in Switzerland. J. Lipid Res. 35:574-583.
3. Hobbs, H. H., M. S. Brown, und J. L. Goldstein. 1992. Molecular genetics of the LDL receptor gene in familial hypercholesterolemia. Hum. Mutat. 1:445-466.
4. Sambrook, J., E. F. Fritsch, und T. Maniatis. 1989. Molecular Cloning. A Laboratory Manual. Cold Spring Harbor Laboratory Press. Second Edition.:
5. Hixson, J. E. und D. T. Vernier. 1990. Restriction isotyping of human apolipoprotein E by gene amplification and cleavage with Hha I. J. Lipid Res. 31:545-548.
6. Ruzicka, V., W. März, A. Russ, und W. Gross. 1992. Apolipoprotein B(Arg3500 to Gln) allele specific polymerase chain reaction: large-scale screening of pooled blood samples. J. Lipid Res. 33:1563-1567.
7. Schuster, H., G. Rauh, S. Müller, C. Kel-1er, G. Wolfram, und N. Zöllner. 1992. Allele-specific and asymmetric polymerase chain reaction amplification in combination: a one step polymerase chain reaction protocol for rapid diagnosis of familial defective apolipoprotein B-100. Anal. Biochem. 204:22-25.
8. Hansen, P. S., N. Rüdiger, A. Tybjaerg-Hansen, O. Faergeman, und N. Gregersen. 1991. Detection of the apoB-3500 mutation (glutamine for arginine) by gene amplification and cleavage with MspI. J. Lipid Res. 32:1229-1233.
9. Miserez, A. R., H. Schuster, N. Chiodetti, und U. Keller. 1993. Polymorphic Haplotypes and recombination rates at the LDL receptor gene locus in subjects with and without familial hypercholesterolemia who are from different populations. Am. J. Hum. Genet. 52:808-826.
10. Miserez, A. R. und U. Keller. 1995. Differences in the phenotypic characteristics of subjects with familial defective apolipoprotein B-100 and familial hypercholesterolemia. Arterioscler. Thromb. Vasc. Biol. 15:1719-1729.
11. Hua, X., J. Sakai, Y. K. Ho, J. L. Goldstein, und M. S. Brown. 1995. Hairpin orientation of sterol regulatory element-binding protein-2 in cell membranes as determined by protease protection. J. Biol. Chem. 270:29422-29427.
12. Hua, X., C. Yokoyama, J. Wu, M. R. Briggs, M. S. Brown, J. L. Goldstein, und X. Wang. 1993. SREBP-2, a second basic-helix-loop-helix-leucine zipper protein that stimulates transcription by binding to a sterol regulatory element. Proc. Natl. Acad. Sci. USA 90:11603-11607.
13. Oberhänsli, I., D. Pometta, H. Micheli, L. Raymond, und A. Suenram. 1982. Lipid, lipoprotein and apo-A and apo-B lipoprotein distribution in Italian and Swiss schoolchildren. The Geneva Survey. Pediatr. Res. 16:665-669.
14. Burnand, B., V. Wietlisbach, W. Riesen, G. Noseda, M. Barazzoni, M. Rickenbach, und F. Gutzwiller. 1993. Lipides sanguins dans la population suisse: enquete MONICA 1988-89. Schweiz. Med. Wschr. 123(Suppl.48):29-37.
15. Tontonoz, P., J. B. Kim, R. A. Graves, und B. M Spiegelman. 1993. ADD1: a novel helix-loop-helix transcription factor associated with adipocyte determination and differentiation. Mol. Cell Biol. 13:4753-4759.
16. Yokoyama, C., X. Wang, M. R. Briggs, A. Admon, J. Wu, X. Hua, und J. L. Goldstein. 1993. SREBP-1, a basic-helix-loop-helix-leucine zipper protein that controls transcription of the low density lipoprotein receptor gene. Cell 75:187-197.
17. Kan, H. Y., P. Pissios, J. Chambaz, und V. I. Zannis. 1999. DNA binding specificity and transactivation properties of SREBP-2 bound to multiple sites on the human apoA-II promoter. Nucleic Acids Res. 27(4):1104-1117.
18. Sakai, J., A. Nohturfft, J. L. Goldstein, und M. S. Brown. 1998. Cleavage of Sterol Regulatory Element-binding Proteins (SREBPs) at Site-1 Requires Interaction with SREBP Cleavage-activating Protein. (Evidence from in vivo competition studies). J. Biol. Chem. 273(10):5785-5793.
19. Brown, M. S. und J. L. Goldstein. 1997. The SREBP Pathway: Regulation of Cholesterol Metabolism by Proteolysis of a membrane-bound transcription factor. Cell 89:331-340.
20. Hua, X., J. Wu, J.L. Goldstein, M.S. Brown, und H.H. Hobbs. 1995. Structure of the human gene encoding sterol regulatory element binding protein-1 (SREBF1) and localization of SREBF1 and SREBF2 to chromosomes 17p11.2 and 22q13. Genomics 25:667-673.
21. Miserez, A.R., G. Cao, L. C. Probst, und H.H. Hobbs. 1997. Structure of the human gene encoding sterol regulatory element binding protein 2 (SREBF2). Genomics 40:31-40.

## Patentansprüche

1. Verfahren zur Erkennung einer erhöhten oder erniedrigten Sensitivität auf HIV-Therapieverfahren resp. deren Nebenwirkungen und/oder eines erhöhten Risikos für den Ausbruch von Hyperlipidämie und/oder Alzheimer Krankheit und/oder eines erhöhten Mortalitätsrisikos, **dadurch gekennzeichnet, dass** nach Blut- resp. Gewebeentnahme, im Blut resp. Gewebe im Exon 18c von SREBP-1 der Polymorphismus G1028G durch Detektion des Nukleotids G anstelle des Nukleotids C an der Position 10 in der Sequenz 5' GCACCTAGGCAAAGGCTTC 3' (Seq. Id. Nr. 1), an Aminosäureposition 1028 (G1028G), nachgewiesen wird.

2. Verfahren gemäss Anspruch 1, **dadurch gekennzeichnet, dass** die HIV Therapieverfahren die Therapie mit Proteasehemmern beinhalten.

3. Verfahren gemäss Anspruch 1 oder 2, **dadurch gekennzeichnet, dass** der Polymorphismus eine Erkennungssequenz für eine innerhalb des Polymorphismus liegende XmnI Schnittstelle aufweist und dass die Untersuchung unter Verwendung dieser Erkennungssequenz erfolgt.

4. Verfahren gemäss einem der Ansprüche 1 - 3, **dadurch gekennzeichnet, dass** nach Blut- resp. Gewebeentnahme und DNA-Extraktion mindestens ein Teilstück eines Exons 18c von SREBP-1, das den Polymorphismus G1028G enthält, unter Verwendung zweier Oligonukleotidsequenzen amplifiziert wird und dass das Produkt der Amplifikation einer Verdauung mit dem Restriktionsenzym XmnI oder einer Denaturierung unterworfen wird und dass die Verdauungsresp. Denaturierungsprodukte elektrophoretisch aufgetrennt werden.

5. Verfahren gemäss Anspruch 4, **dadurch gekennzeichnet, dass** mindestens eine der Oligonukleotidsequenzenen im Intronbereich angesiedelt ist, der dem Exon 18c benachbart ist, in dem der Polymorphismus existiert.

6. Verfahren gemäss einem der Ansprüche 4 oder 5, **dadurch gekennzeichnet, dass** die Oligonukleotidsequenzen ausgewählt sind aus den folgenden Paaren oder damit unter stringenten Bedingungen hybridisierenden Sequenzen:
**S1.18cF** (Seq. Id. Nr. 9): 5'-TTATTTATAATCTGGGTTTTGTGTC-3' und
**S1.18cR** (Seq. Id. Nr. 10): 5'-GGGAAGAGCTAAGTTAAAAGTTGTG-3' oder
***EcoR* I.S1.18cF** (Seq. Id. Nr. 11): 5'- CGGAATTCTGAAATTATTTATAATCTGGGTTTTGTGTC -3'und
***EcoR* I.S1.18cR** (Seq. Id. Nr. 12): 5'-CGGAATTCATCGGGGAAGAGCTAAGTTAAAAGTTGTG-3'.

7. Verfahren gemäss einem der vorangehenden Ansprüche, **dadurch gekennzeichnet, dass** die Hyperlipidämie Hypercholesterinämie ist.

8. Oligonukleotidsequenz zum Nachweis eines Polymorphismus, **dadurch gekennzeichnet, dass** sie die Sequenz 5'GCACCTAGGCAAAGGCTTC 3'(Seq. Id. Nr. 1) des SREBP-1 Gen im Exon 18c umfasst, in der an der Position 10 das Nukleotid C durch das Nukleotid G ersetzt ist.

9. DNA- und/oder RNA-Chip, **dadurch gekennzeichnet, dass** er mindestens die Sequenz 5'GCACCTAGGCAAAGGCTTC 3'(Seq. Id. Nr. 1) des SREBP-1 Gen im Exon 18c und die Sequenz gemäss Anspruch 8 aufweist.

10. DNA- und/oder RNA-Chip gemäss Anspruch 9, **dadurch gekennzeichnet, dass** er zusätzlich mindestens die Sequenz 5' CTGCTGCCGCCAACCTACA 3' (Seq. Id. Nr. 5) des SREBP-2 Gen im Exon 10 und diese Sequenz, in der an der Position 10 das Nukleotid C durch das Nukleotid G ersetzt ist, aufweist.

11. Verwendung einer Sequenz gemäss Anspruch 8 oder eines Chips gemäss Anspruch 9 oder 10 zur Bestimmung eines erhöhten oder verminderten Risikos für das Auftreten von Nebenwirkungen bei der HIV-Therapie, insbesondere der Therapie mit Proteasehemmern.

12. Verwendung einer Sequenz gemäss Anspruch 8 oder eines Chips gemäss Anspruch 9 oder 10 als Marker zur Bestimmung eines erhöhten oder verminderten Risikos für den Ausbruch der Alzheimer-Krankheit und/oder Hyperlipidämie.

13. Verwendung einer Sequenz gemäss Anspruch 8 oder eines Chips gemäss Anspruch 9 oder 10 zur Prüfung der Eignung einer Behandlung mit speziellen Medikamenten für eine Krankheit ausgewählt aus der Gruppe umfassend Hypercholesterinämie, Alzheimer-Krankheit und HIV, oder für das Wirkstoffscreening.

14. Verwendung einer Sequenz gemäss Anspruch 8 oder eines Chips gemäss Anspruch 9 oder 10 zur Bestimmung eines erhöhten oder verminderten Mortalitätsrisikos.

## Claims

1. A method for the detection of an increased or decreased sensitivity to a method of HIV therapy or their side effects, respectively, and/or an increased risk for hyperlipidemia and/or Alzheimer's disease and/or an increased mortality risk, **characterized in that** after taking a blood or a tissue sample, respectively, the polymorphism G1028G in Exon 18c of SREBP-1 is verified in said blood or tissue via detection of the nucleotide G instead of nucleotide C at position 10 in the sequence 5' GCACCTAGGCAAAGGCTTC 3' (Seq. Id. No. 1), at amino acid position 1028 (G1028G).

2. The method of claim 1, **characterized in that** the method of HIV therapy comprises the therapy with protease inhibitors.

3. The method of claim 1 or 2, **characterized in that** the polymorphism shows a recognition sequence for a XmnI cleavage site lying within said polymorphism and that the examination is done using said recognition sequence.

4. The method of anyone of claims 1 - 3, **characterized in that** after taking blood or tissue, respectively, and DNA extraction at least a fragment of an exon 18c of SREBP-1 comprising the polymorphism G1028G is amplified using two oligonucleotide sequences and that the product of the amplification is subjected to a digestion with the restriction enzyme XmnI or a denaturation and that the digestion products or denaturation products, respectively, are separated electrophoretically.

5. The method of claim 4, **characterized in that** at least one of the oligonucleotide sequences is located in the intron region which is adjacent to the exon 18c where said polymorphism exists.

6. The method of one of claims 4 or 5, **characterized in that** the oligonucleotide sequences are selected from the following pairs or from sequences which hybridize therewith under stringent conditions:
**S1.18cF** (Seq. Id. No. 9): 5'-TTATTTATAATCTGGGTTTTGTGTC-3' and
**S1.18cR** (Seq. Id. No. 10): 5'-GGGAAGAGCTAAGTTAAAAGTTGTG-3' or
***EcoR* I.S1.18cF** (Seq. Id. No. 11): 5'- CGGAATTCTGAAATTATTTATAATCTGGGTTTTGTGTC -3' and
***EcoR* I.S1.18cR** (Seq. Id. No. 12): 5'-CGGAATTCATCGGGGAAGAGCTAAGTTAAAAGTTGTG-3'.

7. The method of one of the preceding claims, **characterised in that** the hyperlipidemia is hypercholesterolemia.

8. An oligonukleotide sequence for the determination of a polimorphism, **characterised in that** it comprises the sequence 5'GCACCTAGGCAAAGGCTTC 3'(Seq. Id. No. 1) of the SREBP-1 gene in exon 18c, wherein at position 10 the nucleotide C is replaced by nucleotide G.

9. A DNA and/or RNA chip, **characterized in that** it comprises at least the sequence 5'GCACCTAGGCAAAGGCTTC 3'(Seq. Id. No. 1) of the SREBP-1 gene in exon 18c and the sequence of claim 8.

10. The DNA and/or RNA chip of claim 9, **characterized in that** it additionally comprises at least the sequence 5' CTGCTGCCGCCAACCTACA 3' (Seq. Id. No. 5) of the SREBP-2 gene in exon 10 and this sequence wherein at position 10 the nucleotide C is replaced by the nucleotide G.

11. Use of a sequence of claim 8 or a chip of claim 9 or 10 for the determination of an increased or reduced risk for the occurrence of side effects associated with HIV therapy, in particular the therapy with protease inhibitors.

12. Use of a sequence of claim 8 or a chip of claim 9 or 10 as marker for the determination of an increased or reduced risk for the outbreak of Alzheimer's disease and/or hyperlipidemia.

13. Use of a sequence of claim 8 or a chip of claim 9 or 10 for investigating the suitability of a treatment with specific medicaments for a disease selected from the group comprising hypercholesterinemia, Alzheimer's disease and HIV or for the drug screening.

14. Use of a sequence of claim 8 or a chip of claim 9 or 10 for the determination of an enhanced or reduced mortality risk.

## Revendications

1. Procédé pour reconnaître une sensibilité accrue ou amoindrie à des procédés de thérapie du VIH ou à ses effets secondaires et/ou à un risque accru d'apparition d'une hyperlipidémie et/ou d'une maladie d'Alzheimer et/ou à un risque accru de mortalité, **caractérisé en ce qu'**après un prélèvement de sang ou de tissu on met en évidence, en la position d'acide aminé 1028 (G1028G), dans l'Exon 18c de SREBP-1 le polymorphisme G1028G en détectant le nucléotide G au lieu du nucléotide C en la position 10 dans la séquence 5' GCACCTAGGCAAAGGCTTC 3' (identification de séquence n° 1).

2. Procédé suivant la revendication 1, **caractérisé en ce que** le procédé de thérapie du VIH comporte la thérapie par des inhibiteurs de protéase.

3. Procédé suivant la revendication 1 ou 2, **caractérisé en ce que** le polymorphisme comporte une séquence de reconnaissance d'une interface XmnI se trouvant au sein du polymorphisme et **en ce que** la recherche s'effectue en utilisant cette séquence de reconnaissance.

4. Procédé suivant l'une des revendications 1 à 3, **caractérisé en ce qu'**après prélèvement de sang ou de tissu et extraction de l'ADN, on amplifie au moins une partie d'un Exon 18c de SREBP-1 qui contient le polymorphisme G1028G en utilisant deux séquences d'oligonucléotide et **en ce que** l'on soumet le produit de l'amplification à une digestion avec l'enzyme XmnI de restriction ou à une dénaturation et **en ce que** l'on sépare par électrophorèse les produits de digestion ou de dénaturation.

5. Procédé suivant la revendication 4, **caractérisé en ce qu'**au moins l'une des séquences d'oligonucléotide est établie dans la partie d'un tronc qui est voisine de l'Exon 18c, dans lequel le polymorphisme existe.

6. Procédé suivant l'une des revendications 4 ou 5, **caractérisé en ce que** l'on choisit les séquences d'oligonucléotide dans les paires suivantes ou dans des séquences s'hybridant avec elles dans des conditions strictes
S1.18cF (Identification de séquence N° 9) : 5'-TTATTTATAATCTGGGTTTTGTGTC-3' et
S1.18cR (Identification de séquence N° 10) : 5'-GGGAAGAGCTAAGTTAAAAGTTGTG-3' ou
EcoR I.S1.18cF (Identification de séquence N° 11) : 5'-CGGAATTCTGAAATTATTTATAATCTGGGTTTTGTGTC-3' et
EcoR I.S1.18cR (Identification de séquence N°12) : 5'-CGGAATTCATCGGGGAAGAGCTAAGTTAAAAGTTGTG-3'.

7. Procédé suivant l'une des revendications précédentes, **caractérisé en ce que** l'hyperlipidémie est l'hypercholestérinémie.

8. Séquence d'oligonucléotide pour mettre en évidence un polymorphisme, **caractérisé en ce qu'**elle comprend la séquence 5' GCACCTAGGCAAAGGGCTTC 3' (Identification de séquence n° 1) du gène SREBP-1 dans l'Exon 18c, dans laquelle en la position 10 le nucléotide C est remplacé par le nucléotide G.

9. Site d'ADN et/ou d'ARN, **caractérisé en ce qu'**il a au moins la séquence 5' GCACCTAGGCAAAGGGCTTC 3' (Identification de séquence n° 1) du gène SREBP-1 dans l'Exon 18c et la séquence suivant la revendication 8.

10. Site d'ADN et/ou d'ARN suivant la revendication 9, **caractérisé en ce qu'**il a, en outre, au moins la séquence 5' CTGCTGCCGCCAACCTACA 3' (Identification de séquence n° 5) du gène SREBP-2 dans l'Exon 10 et cette séquence dans laquelle, en la position 10, le nucléotide C est remplacé par le nucléotide G.

11. Utilisation d'une séquence suivant la revendication 8 ou d'un site suivant la revendication 9 ou 10 pour déterminer un risque accru ou moindre de l'apparition d'effets secondaires dans la thérapie du VIH, notamment dans la thérapie par des inhibiteurs de protéase.

12. Utilisation d'une séquence suivant la revendication 8 ou d'un site suivant la revendication 9 ou 10 comme marqueur pour la détermination d'un risque accru ou moindre de l'apparition de la maladie d'Alzheimer et/ou de l'hyperlipidémie.

13. Utilisation d'une séquence suivant la revendication 8 ou d'un site suivant la revendication 9 ou 10 pour contrôler le bien fondé d'un traitement par des médicaments spéciaux pour une maladie choisie dans le groupe comprenant l'hypercholestérinémie, la maladie d'Alzheimer et le VIH ou pour le criblage de principes actifs.

14. Utilisation d'une séquence suivant la revendication 8 ou d'un site suivant la revendication 9 ou 10 pour la détermination d'un risque accru ou moindre de mortalité.


### SEQUENZPROTOKOLL

<110> Miserez, André R.
<120> DNA-Polymorphismen in Stero-Regulator Element-Bindenden Proteinen
<130> Seq. Listing zu 02280PC
<140>
   <141>
<150> CH 1277/99
   <151> 1999-07-09
<160> 20
<170> PatentIn Ver. 2.1
<210> 1
   <211> 19
   <212> DNA
   <213> Homo sapiens
<220>
   <221> CDS
   <222> (2)..(19)
<400> 1
   g cac cta ggc aaa ggc ttc                                          19
     His Leu Gly Lys Gly Phe
       1               5
<210> 2
   <211> 6
   <212> PRT
   <213> Homo sapiens
<400> 2
   His Leu Gly Lys Gly Phe
     1               5
<210> 3
   <211> 19
   <212> DNA
   <213> Homo sapiens
<220>
   <221> CDS
   <222> (2)..(19)
<400> 3
   g cac cta ggg aaa ggc ttc 19
   His Leu Gly Lys Gly Phe
   1 5
<210> 4
   <211> 6
   <212> PRT
   <213> Homo sapiens
<400> 4
   His Leu Gly Lys Gly Phe
   1 5
<210> 5
   <211> 19
   <212> DNA
   <213> Homo sapiens
<400> 5
   ctgctgccgc caacctaca 19
<210> 6
   <211> 7
   <212> PRT
   <213> Homo sapiens
<400> 6
   Ala Ala Ala Ala Asn Leu Gln
   1 5
<210> 7
   <211> 19
   <212> DNA
   <213> Homo sapiens
<400> 7
   ctgctgccgg caacctaca 19
<210> 8
   <211> 7
   <212> PRT
   <213> Homo sapiens
<400> 8
   Ala Ala Ala Gly Asn Leu Gln
   1 5
<210> 9
   <211> 25
   <212> DNA
   <213> Künstliche Sequenz
<220>
   <223> Description of Artificial Sequence: Primer
<220>
   <223> Beschreibung der künstlichen Sequenz: PCR Primer
<400> 9
   ttattaataa tctgggtttt gtgtc 25
<210> 10
   <211> 25
   <212> DNA
   <213> Künstliche Sequenz
<220>
   <223> Description of Artificial Sequence: Primer
<220>
   <223> Beschreibung der künstlichen Sequenz: PCR Primer
<400> 10
   gggaagagct aagttaaaag ttgtg 25
<210> 11
   <211> 38
   <212> DNA
   <213> Künstliche Sequenz
<220>
   <223> Description of Artificial Sequence: Primer
<220>
   <223> Beschreibung der künstlichen Sequenz: PCR Primer
<400> 11
   cggaattctg aaattattta taatctgggt tttgtgtc 38
<210> 12
   <211> 37
   <212> DNA
   <213> Künstliche Sequenz
<220>
   <223> Description of Artificial Sequence: Primer
<220>
   <223> Beschreibung der künstlichen Sequenz: PCR Primer
<400> 12
   cggaattcat cggggaagag ctaagttaaa agttgtg 37
<210> 13
   <211> 25
   <212> DNA
   <213> Künstliche Sequenz
<220>
   <223> Description of Artificial Sequence: Primer
<220>
   <223> Beschreibung der künstlichen Sequenz: PCR Primer
<400> 13
   gccagtgacc attaacacct tttga 25
<210> 14
   <211> 30
   <212> DNA
   <213> Künstliche Sequenz
<220>
   <223> Description of Artificial Sequence: Primer
<220>
   <223> Beschreibung der künstlichen Sequenz: PCR Primer
<400> 14
   tcgtcttcaa agcctgcctc agtggctggc 30
<210> 15
   <211> 33
   <212> DNA
   <213> Künstliche Sequenz
<220>
   <223> Description of Artificial Sequence: Primer
<220>
   <223> Beschreibung der künstlichen Sequenz: PCR Primer
<400> 15
   cggaattcgc cagtgaccat taacaccttt tga 33
<210> 16
   <211> 33
   <212> DNA
   <213> Künstliche Sequenz
<220>
   <223> Description of Artificial Sequence: Primer
<220>
   <223> Beschreibung der künstlichen Sequenz: PCR Primer
<400> 16
   cggaattctg cagcaagcca gtcatcagca gct 33
<210> 17
   <211> 33
   <212> DNA
   <213> Künstliche Sequenz
<220>
   <223> Description of Artificial Sequence: Primer
<220>
   <223> Beschreibung der künstlichen Sequenz: PCR Primer
<400> 17
   cggaattctg gtctcactgt gttttcactc atc 33
<210> 18
   <211> 33
   <212> DNA
   <213> Künstliche Sequenz
<220>
   <223> Description of Artificial Sequence: Primer
<220>
   <223> Beschreibung der künstlichen Sequenz: PCR Primer
<400> 18
   cggaattcgc cagggctgac aagccttttc tca 33
<210> 19
   <211> 46
   <212> DNA
   <213> Künstliche Sequenz
<220>
   <223> Description of Artificial Sequence: Primer
<220>
   <223> Beschreibung der künstlichen Sequenz: PCR Primer
<400> 19
   gccagaggag attttgcagc tgctgccggc aacctacaaa cctgcc 46
<210> 20
   <211> 46
   <212> DNA
   <213> Künstliche Sequenz
<220>
   <223> Description of Artificial Sequence: Primer
<220>
   <223> Beschreibung der künstlichen Sequenz: PCR Primer
<400> 20
   ggcaggtttg taggttgccg gcagcagctg caaaatctcc tctggc 46